(19) Europäisches Patentamt
European Patent Office
Office européen des brevets

(11) **EP 4 745 133 A1**

(12) **EUROPEAN PATENT APPLICATION**
published in accordance with Art. 153(4) EPC

(43) Date of publication:
**20.05.2026 Bulletin 2026/21**

(21) Application number: **24840142.4**

(22) Date of filing: **12.07.2024**

(51) International Patent Classification (IPC):
***C07D 401/14*** *(2006.01)* ***A61K 31/4439*** *(2006.01)*
***A61P 25/28*** *(2006.01)* ***A61P 9/00*** *(2006.01)*
***A61P 37/00*** *(2006.01)* ***A61P 1/16*** *(2006.01)*

(52) Cooperative Patent Classification (CPC):
**A61K 31/4439; A61P 1/16; A61P 9/00; A61P 25/28; A61P 37/00; C07D 401/14**

(86) International application number:
**PCT/KR2024/010056**

(87) International publication number:
**WO 2025/014332 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **12.07.2023 KR 20230090746**

(71) Applicant: **Korea Research Institute of Chemical Technology**
**Daejeon 34114 (KR)**

(72) Inventors:
- **LEE, Kwang Ho**
  **Daejeon 34114 (KR)**
- **SONG, Jin Sook**
  **Daejeon 34114 (KR)**
- **KIM, Seong Hwan**
  **Daejeon 34114 (KR)**
- **AHN, Sunjoo**
  **Daejeon 34114 (KR)**
- **CHAE, Chong Hak**
  **Daejeon 34114 (KR)**
- **DUGGIRALA, Krishna Babu**
  **Daejeon 34114 (KR)**
- **LEE, Yujin**
  **Gimcheon-si Gyeongsangbuk-do 39589 (KR)**
- **JI, Sang Hee**
  **Anseong-si Gyeonggi-do 17558 (KR)**
- **JANG, Jiyoon**
  **Bucheon-si Gyeonggi-do 14549 (KR)**

(74) Representative: **Potter Clarkson**
**Chapel Quarter**
**Mount Street**
**Nottingham NG1 6HQ (GB)**

# (54) TRIAZOLE COMPOUND HAVING NOVEL RING STRUCTURE, METHOD FOR PREPARING SAME, AND USE THEREOF

(57) The present invention relates to a triazole compound having a novel ring structure; a method for preparing same; and a use thereof. The triazole compound having a novel ring structure according to the present invention exhibits an excellent ability to inhibit ASK1 and inhibits the apoptosis of neurons and thus can be used for preventing or treating ASK1-related diseases including neurodegenerative diseases, cardiovascular diseases, autoimmune diseases, and liver diseases.

[FIG. 2]

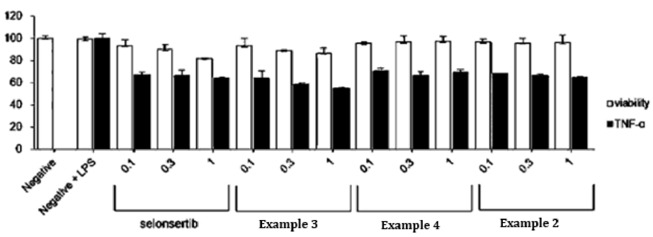


EP 4 745 133 A1

## Description

## Technical Fields

The present invention relates to a triazole compound with a novel ring structure, a method for preparing the same, and use thereof.

## Background Technology

ASK1(Apoptosis signal-regulating kinase 1; ASK1) is a c-Jun N-terminal protein kinase (JNK) and a stress-responsive mitogen-activated protein kinase (mitogen-activated protein kinase; MAP3K), also known as MAP3K5. ASK1 is a central regulator of apoptosis and is known to participate in several stress-induced and receptor-mediated apoptosis pathways triggered by various forms of stress, including oxidative stress, reactive oxygen species (ROS), endoplasmic reticulum (ER) stress and unfolding protein responses (UPRs), and mitochondrial stress.

In addition, ASK1 plays an essential role not only in the apoptotic pathway, but also in inflammatory and innate immune responses, including cytokine responses and cell differentiation. Phosphorylation of the ASK1 protein can lead to apoptosis or other cellular responses, depending on the cell type. In particular, ASK1 activation and signaling have been reported to play an important role in a wide range of diseases, including neurodegenerative, cardiovascular, inflammatory, autoimmune, and metabolic diseases. In addition, ASK1 is known to be involved in mediating organ damage due to ischemia and reperfusion of the heart, brain, and kidneys (Zhang et al. J Clin Invest.2003; 111(12): 1933-1943.).

On the other hand, Parkinson's disease is a neurodegenerative disease that occurs mainly in the elderly. Parkinson's disease causes dopaminergic neurons in the substantia nigra in the middle of the brain to die, causing behavioral impairment. In addition, Lewy bodies, which are formed by the deposition of alpha synuclein proteins in various parts of the brain and peripheral nerves, are the main neuropathological features of Parkinson's disease patients. Typical symptoms of Parkinson's disease include Bradykinesia, which is an abnormal slowdown in body movements, tremors in the hands and feet, and rigidity that causes stiffness in muscles and joints, and non-motor symptoms including mental disorders and autonomic nervous system dysfunction.

At this time, it has been reported that excessive amounts of ASK1 are closely related to heart disease, inflammatory disease, liver disease, infectious disease, and degenerative brain disease, and the possibility of ASK1 inhibition-based Parkinson's disease treatment is being raised from various angles. When Parkinson's disease is induced by alpha-synuclein pre-formed fibrils (PFFs), the accumulation of phosphorylated alpha-synuclein (p-alpha-synuclein) in the striatum and cerebral cortex is reduced in ASK1-deficient mice. Furthermore, neuroinflammation was alleviated, which led to behavioral improvements. In addition, the activation of p-ASK1 was observed to be more than four times that of normal in neurons in the substantia nigra region of patients with Parkinson's disease.

Accordingly, in the course of researching ASK1 derivatives, the present inventors completed the present invention by confirming that the new triazole compounds have excellent ASK1 inhibition ability, protect nerve cells, easily penetrate the cell membrane, and are not an effluent transporter.

## Detailed Description Of The Invention

## Technical Challenges

An embodiment of the present invention provides triazole compounds with novel ring structures.

An embodiment of the present invention provides a method for the preparation of triazole compounds with a novel ring structure.

An embodiment of the present invention provides a pharmaceutical composition containing a triazole compound with a novel ring structure as an active ingredient.

An embodiment of the present invention provides a pharmaceutical composition for preventing or treating ASK1-related diseases containing a novel ring-structured triazole compound as an active ingredient.

An embodiment of the present invention provides a method for preventing or treating ASK1-related diseases, comprising the step of administering a novel ring-structured triazole compound to a subject in need thereof.

An embodiment of the present invention provides the use of a novel ring-structured triazole compound to use for preventing or treating ASK1-related diseases.

An embodiment of the present invention provides the use of a novel ring-structured triazole compound for the preparation of a drug for preventing or treating ASK1-related diseases.

**Tools To Solve The Task**

One embodiment of the present invention provides a compound represented by the following Chemical Formula 1, or a stereoisomer, a solvate, a hydrate, or a pharmaceutically acceptable salt thereof:

[Chemical Formula 1]

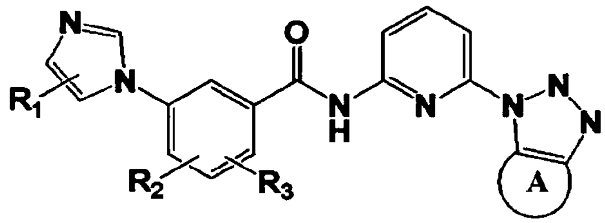

In the above Chemical Formula 1,

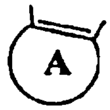

is a substituted or unsubstituted phenyl or substituted or unsubstituted $C_{3-8}$ cycloalkenyl,

wherein, the substituted phenyl and substituted $C_{3-8}$ cycloalkenyl are each independently substituted with $C_{1-6}$ alkyl, $C_{1-6}$ alkoxy or halogen;

$R_1$, $R_2$ and $R_3$ are each independently H, $C_{1-6}$ alkyl, halogen, $C_{3-6}$ cycloalkyl or halo $C_{1-6}$ alkyl.

One embodiment of the present invention provides a method for preparation of a compound represented by Chemical Formula 1 comprising: reacting a compound represented by Chemical Formula 2 with a compound represented by Chemical Formula 3 to prepare a compound represented by Chemical Formula 1, as shown in Reaction Equation 1 below:

[Reaction Equation 1]

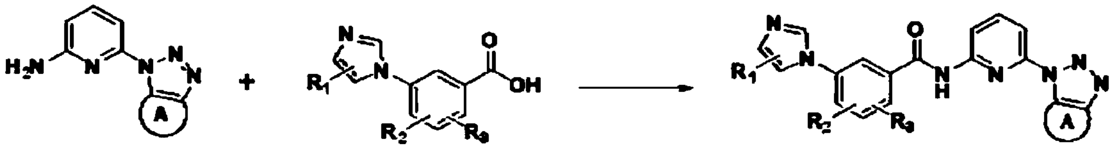

2 3 1

In the above Reaction Equation 1,

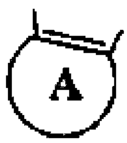

,

$R_1$, $R_2$, and $R_3$ are same as defined in Chemical Formula 1 above.

One embodiment of the present invention provides a pharmaceutical composition for preventing or treating ASK1-related diseases comprising a compound represented by Chemical Formula 1, or a stereoisomer, a solvate, a hydrate, or a pharmaceutically acceptable salt thereof as an active ingredient.

One embodiment of the present invention provides a method for preventing or treating ASK1-related diseases, comprising the steps of administering a pharmaceutical composition comprising a compound represented by Chemical Formula 1, or a stereoisomer, a solvate, a hydrate, or a pharmaceutically acceptable salt thereof to a subject.

One embodiment of the present invention provides a use of a pharmaceutical composition for preventing or treating an ASK1 related disease, wherein the pharmaceutical composition comprises a compound represented by Chemical Formula 1 or a stereoisomer, a solvate, a hydrate, or a pharmaceutically acceptable salt thereof.

One embodiment of the present invention provides the use of a pharmaceutical composition for preventing or treating an ASK1 related disease, wherein the pharmaceutical composition comprises a compound represented by Chemical Formula 1 or a stereoisomer, a solvate, a hydrate, or a pharmaceutically acceptable salt thereof.

## Advantage of The Invention

The triazole compound of the novel ring structure of the present invention exhibits high inhibitory activity against ASK1 and inhibits neuronal cell death, so it can be used for preventing or treating ASK1-related diseases, including neurodegenerative diseases, cardiovascular diseases, and autoimmune diseases. For example, the compound of the present invention can effectively prevent or treat Parkinson's disease by improving behavioral deficits or errors and symptoms of neurological disorders associated with Parkinson's disease, significantly improving dopaminergic neurodegeneration, and reducing microglia. In addition, the compound of the present invention can effectively prevent or treat amyotrophic lateral sclerosis by improving neurological disorders or disease symptoms and reduced grip strength and increasing the survival rate. Furthermore, the compound of the present invention can effectively prevent or treat fibromyalgia by improving the pain caused by fibromyalgia. In addition, the compound of the present invention can improve liver fibrosis caused by non-alcoholic steatohepatitis, thereby effectively preventing or treating non-alcoholic steatohepatitis.

## Brief Description Of The Drawings

FIG. 1 presents the neuroprotection assay showing the effect of the cell protection by % of control.

FIG. 2 shows the cell viability (%) of LPS-treated cells and TNF-$\alpha$ ELISA results.

FIG. 3 is a schematic diagram of an experiment to confirm the efficacy of the drug in an MPTP-induced PD mouse model.

FIG. 4 is a graph showing the results of group-specific behavioral deficit assessment (pole test) by administration of the drug in an MPTP-induced PD mouse model, wherein, a, b, and c represent the rotation time at the top, the column descent time, and the total time spent, respectively. Significance level over G1 - *: $p < 0.05$, **: $p < 0.01$, ***: $p < 0.001$ / Significance level over G2 - #: $p < 0.05$, ##: $p < 0.01$, ###: $p < 0.001$.

FIG. 5a is a graph showing the degree of TH staining of striatum for each group following drug treatment in an MPTP-induced PD mouse model. Significance level over G1 - *: $p < 0.05$, **: $p < 0.01$, ***: $p < 0.001$ / Significance level over G2 - #: $p < 0.05$, ##: $p < 0.01$, ###: $p < 0.001$.

FIG. 5b shows the degree of TH staining of striatum by group in an MPTP-induced PD mouse model.

FIG. 6a is a graph showing the degree of TH staining of substantia nigra by group in an MPTP-induced PD mouse model following drug treatment. Significance level over G1 - *: $p < 0.05$, **: $p < 0.01$, ***: $p < 0.001$ / Significance level over G2 - #: $p < 0.05$, ##: $p < 0.01$, ###: $p < 0.001$.

FIG. 6b shows the degree of TH staining of the substantia nigra by group following drug treatment in an MPTP-induced PD mouse model.

FIG. 7 is a graph confirming the dopamine concentrations by group according to drug treatment in the striatum and substantia nigra of the MPTP-induced PD mouse model. Significance level over G1 - *: $p < 0.05$, **: $p < 0.01$, ***: $p < 0.001$ / Significance level over G2 - #: $p < 0.05$, ##: $p < 0.01$, ###: $p < 0.001$.

FIG. 8 is a schematic diagram of an experiment to confirm the efficacy of the drug in a mouse model of $\alpha$-Syn transformed Parkinson's disease.

FIG. 9 is a graph showing the change in body weight by group following drug treatment in a mouse model of $\alpha$-Syn transformed Parkinson's disease. Significance level over G1 - *: $p < 0.05$, **: $p < 0.01$, ***: $p < 0.001$ / Significance level over G2 - #: $p < 0.05$, ##: $p < 0.01$, ###: $p < 0.001$.

FIG. 10 is a graph of group-specific neurological behavioral scores following drug treatment in a $\alpha$-Syn transformed Parkinson's disease mouse model. Significance level over G1 - *: $p < 0.05$, **: $p < 0.01$, ***: $p < 0.001$ / Significance level over G2 - #: $p < 0.05$, ##: $p < 0.01$, ###: $p < 0.001$.

FIG. 11 is a graph showing the results of group-specific gait error assessment following drug treatment in a $\alpha$-Syn transformed Parkinson's disease mouse model. Significance level over G1 - *: $p < 0.05$, **: $p < 0.01$, ***: $p < 0.001$ /

Significance level over G2 - #: $p < 0.001$, ##: $p < 0.01$, ###: $p < 0.001$.

FIG. 12a is a graph showing the results of the evaluation of the efficacy evaluation of dopaminergic neurodegeneration by group following drug treatment in a α-Syn transformed Parkinson's disease mouse model. Significance level over G1 - *: $p < 0.05$, **: $p < 0.01$, ***: $p < 0.001$ / Significance level over G2 - #: $p < 0.05$, ##: $p < 0.01$, ###: $p < 0.001$.

FIG. 12b is an immunofluorescence analysis image showing the results of the evaluation of the efficacy of improving dopaminergic neurodegeneration by group following drug treatment in a mouse model of α-Syn transformed Parkinson's disease.

FIG. 13a is a graph showing the results of the evaluation of microglial reduction efficacy by group following drug treatment in a mouse model of α-Syn transformed Parkinson's disease. Significance level over G1 - *: $p < 0.05$, **: $p < 0.01$, ***: $p < 0.001$ / Significance level over G2 - #: $p < 0.05$, ##: $p < 0.01$, ###: $p < 0.001$.

FIG. 13b is an immunofluorescence assay image showing the results of the evaluation of microglial reduction efficacy by group following drug treatment in a mouse model of α-Syn transgenic Parkinson's disease.

FIG. 14 is a schematic diagram of an experiment for an experiment to confirm the efficacy of the drug in a mouse model of SOD1 G93A transgenic ALS.

FIG. 15 is a graph of the change in body weight by group following drug treatment in a SOD1 G93A transgenic ALS mouse model. Significance level compared to G1 - *: $p < 0.05$, **: $p < 0.01$, ***: $p < 0.001$.

FIG. 16 is a graph of the group-specific neurological scores following drug treatment in an animal model of SOD1 G93A transformed ALS. Significance level over G1 - *: $p < 0.05$, **: $p < 0.01$, ***: $p < 0.001$ / Significance level over G2 - #: $p < 0.05$, ##: $p < 0.01$, ###: $p < 0.001$.

FIG. 17 is a graph of the change in disease scores by group with drug treatment in a mouse model of SOD1 G93A transgenic ALS. Significance level over G1 - *: $p < 0.05$, **: $p < 0.01$, ***: $p < 0.001$ / Significance level over G2 - #: $p < 0.05$, ##: $p < 0.01$, ###: $p < 0.001$.

FIG. 18 is a graph of the group-specific change in grip strength with drug treatment in a SOD1 G93A transgenic ALS mouse model. Significance level over G1 - *: $p < 0.05$, **: $p < 0.01$, ***: $p < 0.001$ / Significance level over G2 - #: $p < 0.05$, ##: $p < 0.01$, ###: $p < 0.001$.

FIG. 19 is a graph of group-specific survival rate following drug treatment in a SOD1 G93A transgenic ALS mouse model.

FIG. 20 is a schematic diagram of an experiment to confirm the efficacy of the drug in a rat model of Reserpine-induced fibromyalgia.

FIG. 21 is a graph of the results of evaluating the efficacy of pain improvement following drug treatment in a Reserpine-induced fibromyalgia rat model. Significance level over G1 - *: $p < 0.05$, **: $p < 0.01$, ***: $p < 0.001$ / Significance level over G2 - #: $p < 0.05$, ##: $p < 0.01$, ###: $p < 0.001$.

FIG. 22 is a schematic diagram of an experiment to confirm the efficacy of the drug in a mouse model of CDAHFD-induced non-alcoholic steatohepatitis.

FIG. 23a is a graph of the ratio of Picrosirius red stained area in the liver following drug treatment in a mouse model of CDAHFD-induced non-alcoholic steatohepatitis. Significance level over G1 - *: $p < 0.05$, **: $p < 0.01$, ***: $p < 0.001$ / Significance level over G2 - #: $p < 0.05$, ##: $p < 0.01$, ###: $p < 0.001$.

FIG. 23b is an image of the Picrosirius red staining area ratio in the liver following drug treatment in a CDAHFD-induced nonalcoholic steatohepatitis mouse model.

## Mode of Reduction to Practice the Invention

One embodiment of the present invention provides a compound represented by Chemical Formula 1, or a stereoisomer, a solvate, a hydrate, or a pharmaceutically acceptable salt thereof.

[Chemical Formula 1]

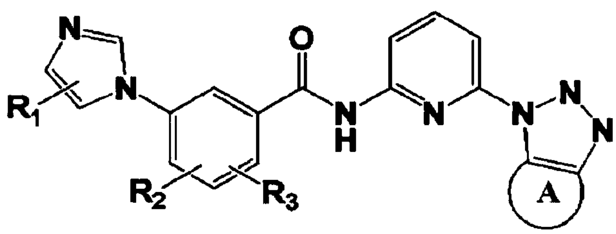

In the above Chemical Formula 1,

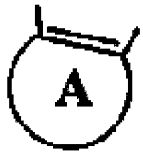

is a substituted or unsubstituted phenyl or substituted or unsubstituted $C_{3-8}$ cycloalkenyl,
wherein, the substituted phenyl and the substituted $C_{3-8}$ cycloalkenyl are each independently substituted with $C_{1-6}$ alkyl, $C_{1-6}$ alkoxy or halogen; and
$R_1$, $R_2$ and $R_3$ are each independently H, $C_{1-6}$ alkyl, halogen, $C_{3-6}$ cycloalkyl or halo $C_{1-6}$ alkyl.

Further, in the above Chemical Formula 1,

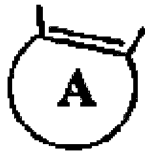

is a substituted or unsubstituted phenyl or substituted or unsubstituted $C_{5-8}$ cycloalkenyl,
wherein, the substituted phenyl and the substituted $C_{3-8}$ cycloalkenyl are each independently substituted with $C_{1-3}$ alkyl, $C_{1-3}$ alkoxy or halogen; and
$R_1$, $R_2$ and $R_3$ are each independently H, $C_{1-3}$ alkyl, halogen, $C_{3-6}$ cycloalkyl or halo $C_{1-3}$ alkyl.

Further, in the above Chemical Formula 1,

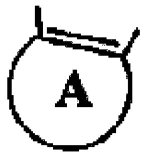

is phenyl or $C_{5-8}$ cycloalkenyl containing a bond;
$R_1$ is $C_{3-6}$ alkyl;
$R_2$ is $C_{1-3}$ alkyl; and
$R_3$ is halogen.

Further, in the above Chemical Formula 1,

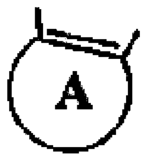

is $C_{5-8}$ cycloalkenyl containing a phenyl or a double bond;
$R_1$ is cyclopropyl;
$R_2$ is methyl; and
$R_3$ is fluorine.

The term used herein, "cycloalkenyl" comprises an arbitrary stable cyclic hydrocarbon group, which comprises one or more unsaturated carbon-carbon double bonds at any location of the ring, may be mono or multi substituted, and may be monovalent, bivalent, or multivalent. For the purposes of this specification, it is a bivalent fused to triazole. An example of an embodiment containing one of these double bonds of cycloalkenyl groups may include cyclopentenyl groups, cyclohexenyl groups, cycloheptenyl groups, and cyclooctenyl groups.
The term "alkyl" used in this specification refers to fully saturated hydrocarbon groups in a straight or branched

chain, and examples of alkyls include methyl (Me), ethyl (Et), propyl (such as n-profile or isopropyl group), butyl (such as n-butyl, isobutyl, or s-butyl, t-butyl), and pentyl (such as n-pentyl, isopentyl group, or neopentyl group).

The term "haloalkyl" as used in this specification includes an alkyl substituted with one or more halogens, single substituted (such as $-CH_2F$) or multiple substituted (e.g., $-CF_3$).

An example of a compound represented by Chemical Formula 1 according to the present invention includes the following compounds:

<1> N-(6-(1H-benzo[d][1,2,3]triazole-1-yl)pyridine-2-day)-5-(4-cyclopropyl-1H-imidazole-1-day)-2-fluoro-4-methylbenzamide;

<2> 5-(4-cyclopropyl-1H-imidazole-1-day)-N-(6-(5,6-dihydrocyclopenta[d][1,2,3]triazole-1(4H)-day)pyridine-2-day)-2-fluoro-4-methylbenzamide;

<3> 5-(4-cyclopropyl-1H-imidazole-1-day)-2-fluoro-4-methyl-N-(6-(4,5,6,7-tetrahydro-1H-benzo[d][1,2,3]triazole-1-day)pyridine-2-day)methylbenzamide;

<4> 5-(4-cyclopropyl-1H-imidazole-1-yl)-2-fluoro-4-methyl-N-(6-(5,6,7,8-tetrahydrocyclohepta[d][1,2,3]triazole-1(4H)-yl)pyridine-2-yl)benzamide; and

<5> 5-(4-cyclopropyl-1H-imidazole-1-yl)-2-fluoro-N-(6-(4,5,6,7,8,9-hexahydro-1H-cycloocta[d][1,2,3]triazole-1-yl) pyridine-2-yl)-4-methylbenzamide.

One embodiment of the present invention provides a method for preparation of a compound represented by Chemical Formula 1 comprising: reacting a compound represented by Chemical Formula 2 with a compound represented by Chemical Formula 3 to prepare a compound represented by Chemical Formula 1, as shown in Reaction Equation 1 below:

[Reaction Equation 1]

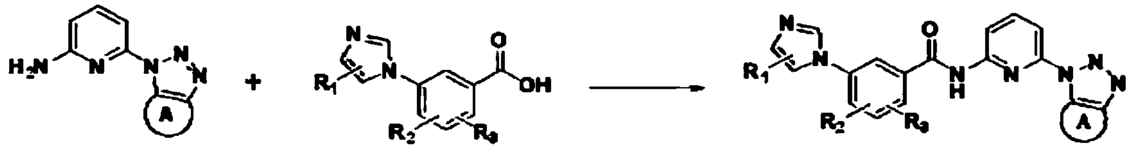

2 3 1

In the above Reaction Equation 1,

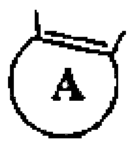

,

$R_1$, $R_2$, and $R_3$ are same as defined in Chemical Formula 1 above.

The above reaction can be performed under the conditions of organic solvents such as DMF (dimethyl formamide) and NMP (N-methyl-2-pyrrolidone), and catalyst substances such as DMAP (4-dimethylaminopyridine) can be used.

Coupling agents such as HATU (Hexafluorophosphate Azabenzotriazole Tetramethyl Uronium) and T3P (propanephosphonic acid anhydride) can be used for the above reactions.

The above reactions can be performed at 0°C to 50°C. For example, the reaction can be performed at 0°C to 40°C, 0°C to 30°C, 10°C to 50°C, 10°C to 40°C, 10°C to 30°C, 20°C to 50°C, 20°C to 40°C or 20°C to 30°C.

The reaction can be performed for 0.1 h to 48 h. For example, the reaction can run for 0.1 hour to 36 hours, 0.1 hours to 24 hours, 0.5 hours to 48 hours, 0.5 hours to 36 hours, 0.5 hours to 24 hours, 1 hour to 48 hours, 1 hour to 36 hours, 1 hour to 24 hours, 2 hours to 48 hours, 2 hours to 36 hours, 2 hours to 24 hours, It can be performed for 3 hours to 48 hours, 3 hours to 36 hours, 3 hours to 24 hours, 4 hours to 48 hours, 4 hours to 36 hours, 4 hours to 24 hours, 6 hours to 48 hours, 6 hours to 36 hours, or 6 hours to 24 hours.

The compound represented by Chemical Formula 1 of the present invention can be used in the form of a pharmaceutically acceptable salt, and an acid salt formed by a pharmaceutically acceptable free acid is useful as a salt. Acid salts are obtained from inorganic acids such as hydrochloric acid, nitric acid, phosphoric acid, sulfuric acid,

hydrobromide acid, hydroiodic acid, nitrous acid, phosphorous acid, non-toxic organic acids such as aliphatic mono and dicarboxylate, phenyl-substituted alkanoates, hydroxy alkanoates and alkandioates, aromatic acids, aliphatic and aromatic sulfonic acids, etc., and organic acids such as trifluoroacetic acid, acetate, benzoic acid, citric acid, lactic acid, maleic acid, gluconic acid, methanesulfonic acid, 4-toluene sulfonic acid, tartaric acid, fumaric acid, etc. These pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suverate, sevacate, fumarate, maliate, butyne-1, 4-dioate, hexane-1,6-dioate, benzoate, chlorobenzoate, methyl benzoate, dinitrobenzoate, hydroxybenzoate, methoxybenzoate, phthalate, terephthalate, benzenesulfonate, toluenesulfonate, chlorobenzene sulfonate, xylene sulfonate, phenyl acetate, phenylpropionate, phenyl butyrate, citrate, lactate, β-hydroxybutyrate, glycolates, malate, tartrate, methane sulfonate, propane sulfonate, naphthalene-1-sulfonate, naphthalene-2-sulfonate, and mandelate.

The acid salt according to the present invention can be prepared by ordinary methods. For example, a derivative of Chemical Formula 1 is dissolved in an organic solvent such as methanol, ethanol, acetone, methylene chloride, acetonitrile, etc., followed by addition of an organic or inorganic acid to form a precipitate, and filtering and drying the precipitate the acid salt or by produced by adding organic or inorganic acids, or distilling the solvent and excess acid by vacuum evaporation to dryness followed by crystallization in an organic acid to produce the acid salts.

In addition, bases can be used to produce pharmaceutically acceptable metal salts. Alkali metals or alkaline earth metal salts are obtained by dissolving compounds in an excess of alkaline metal hydroxides or alkaline earth metal hydroxide solutions, filtering the compound salts, and evaporating and drying the filtrate. In this case, it is suitable for pharmaceutical purposes to manufacture sodium, potassium or calcium salts as metal salts. In addition, the corresponding salt is obtained by reacting alkali metal or alkaline earth metal salt with a suitable negative salt (e.g., silver nitrate).

Further, the present invention includes not only a compound represented by Chemical Formula 1 and a pharmaceutically acceptable salt thereof, but also a solvate, a stereoisomer, a hydrate, etc. that can be prepared therefrom.

The term "hydrate" as used herein means a compound or a salt thereof containing a stoichiometric or non-stoichiometric amount of water combined by a non-covalent intermolecular force. The hydrate of the compound represented by Chemical Formula 1 of the present invention may include stoichiometric or non-stoichiometric amounts of water bonded by non-covalent intermolecular forces. The hydrate may contain more than one equivalent, preferably 1 to 5 equivalents of water. Such hydrate may be prepared by crystallizing a compound represented by Chemical Formula 1 of the present invention, or a stereoisomer, a solvate, a hydrate, or a pharmaceutically acceptable salt thereof, from water or a solvent containing water.

The term "solvate" used herein means a compound or a salt thereof containing a stoichiometric or non-stoichiometric amount of solvent bonded by non-covalent intermolecular forces. Desirable solvents for this include volatile, non-toxic, and/or suitable for administration to humans.

The term "isomer" as used herein means a compound or salt thereof that has the same chemical or molecular formula but is structurally or three-dimensionally different. These isomers include structural isomers and stereoisomers, which include tautomers, and stereoisomers include both optical isomers (enatiomers) and partial stereoisomers (trans, cis) that appear as having one or more asymmetric carbon centers. All of these isomers and their mixtures are also included in the scope of the present invention.

In addition, the above compound, its stereoisomers, its solvents, its hydrates, or its pharmaceutically acceptable salts may inhibit ASK1.

In addition, the above compound, its stereoisomer, its solvent, its hydrate, or its pharmaceutically acceptable salts can inhibit the death of neurons.

One embodiment of the present invention provides a pharmaceutical composition for preventing or treating ASK1-related diseases comprising a compound represented by Chemical Formula 1, or a stereoisomer, a solvent, a hydrate, or a pharmaceutically acceptable salt thereof.

The pharmaceutical composition may further comprise, in addition to the above active ingredients, pharmaceutically acceptable carriers.

The ASK1-related diseases include neurodegenerative diseases, cardiovascular diseases, autoimmune diseases, and liver diseases.

The neurodegenerative diseases include Alzheimer's disease, hippocampal sclerosis, frontotemporal dementia (FTD), frontotemporal degeneration (FTLD), Huntington's disease, corticobasal degeneration, amyotrophic lateral sclerosis, spinal muscular atrophy, motor neuron disease, inclusion body myositis, Parkinson's disease, Lewy body dementia, Lewy body disease, multiple system atrophy, progressive supranuclear palsy, peak disease, Prion disease, traumatic brain injury, ischemic and hemorrhagic stroke, cerebral ischemia, hypoxia, and glutamate neurotoxicity.

The cardiovascular diseases include heart failure, ischemia, recurrent ischemia, myocardial infarction, arrhythmias, acute coronary syndrome, diabetes, atherosclerosis, and intermittent claudication.

The autoimmune diseases include rheumatoid arthritis, fibromyalgia, systemic lupus erythematosus, multiple sclerosis, diabetes, systemic sclerosis, Graves' disease, Guillain-Barré syndrome, myasthenia gravis, psoriasis, Crohn's disease, ulcerative colitis, optic neuritis, and Sjögren's syndrome.

The liver diseases include non-alcoholic fatty liver (NASH), alcoholic fatty liver, liver fibrosis, liver cancer, hepatotoxicity, cholestasis, cirrhosis, liver ischemia, liver abscess, hepatic coma, and liver atrophy.

The compound or the pharmaceutically acceptable salt thereof represented by Chemical Formula 1 may be administered in several formulations, such as oral and parenteral, at the time of clinical administration. When formulated, it is prepared using diluents or excipients such as fillers, bulking agents, binders, wetting agents, disintegrants, and surfactants, which are usually used. Solid formulations for oral administration include tablets, pill agents, acids, granules, capsules, etc., and these solid formulations are prepared by mixing one or more compounds with at least one excipient, such as starch, calcium carbonate, sucrose or lactose, gelatin, etc. In addition to simple excipients, lubricants such as magnesium stearate and talc are also used. Liquid preparations for oral administration include suspensions, liquid containing agents, emulsions, syrups, etc., and in addition to water and liquid paraffin, which are commonly used simple diluents, various excipients, such as wetting agents, sweeteners, air fresheners, and preservatives. Preparations for parenteral administration include sterile aqueous solutions, non-aqueous solvents, suspensions, and emulsions. Non-aqueous solvents and suspension solvents can be used such as propylene glycol, polyethylene glycol, vegetable oils such as olive oil, and injectable esteros such as ethyl oleate.

A pharmaceutical composition comprising a compound represented by Chemical Formula 1 or a pharmaceutically acceptable salt thereof as the active ingredient may be administered parenterally, and parenteral administration may be administered by method of injection subcutaneously, intravenously, intramuscularly, or intrathoracically injected.

In this case, a compound represented by formulation 1 or a pharmaceutically acceptable salt thereof may be mixed with a stabilizer or buffer in water to be prepared as a solution or suspension, and it may be prepared in ampoule or vial unit dosage form. The composition may be sterilized and/or contain adjuvants such as preservatives, stabilizers, hydrating agents or emulsification promoters, salts and/or buffers for osmotic control, and other therapeutically useful substances, and may be formulated according to the usual method of mixing, granulating or coating.

Oral formulations include, for example, tablets, pills, light/soft capsules, liquids, suspensions, emulsifiers, syrups, granules, elixirs, and troches, which contain diluents (e.g., lactose, dextrose, sucrose, mannitol, sorbitol, cellulose, and/or glycine), and lubricants (e.g., silica, talc, stearic acid and its magnesium or calcium salts and/or polyethylene glycol) in addition to the active ingredients. Tablets may contain binders such as magnesium aluminum silicate, starch paste, gelatin, methylcellulose, sodium carboxymethylcellulose, and/or polyvinyl pyrrolidine, etc., and in some cases may contain disintegrating or boiling mixtures and/or absorbents, colorants, flavors, and sweeteners, such as starch, agar, alginate, or its sodium salts, etc.

A compound represented by Chemical Formula 1 of the present invention, a stereoisomer thereof, a solvent thereof, a hydrate thereof, or a pharmaceutical composition thereof, or a pharmaceutical composition comprising thereof, is administered in a "pharmaceutically valid amount". For the purposes of this specification, the term "pharmaceutically valid amount" means an amount sufficient to treat a disease at a reasonable benefit/risk ratio applicable to medical treatment or improvement, and the effective dose level may be determined by individual type and severity, age, sex, activity of the drug, sensitivity to the drug, time of administration, route of administration and rate of elimination, duration of treatment, factors including concurrent drugs used and other factors well known in the field of medicine. For example, available quantities of 0.001 mg/kg to 1000 mg/kg, 0.01 mg/kg to 100 mg/kg or 0.1 to 20 mg/kg or 0.1 to 500 mg/kg are included. The amount of the compound or pharmaceutical composition of the present invention may be selected and carried out by the contractor within an appropriate range.

One embodiment of the present invention provides a functional health food for preventing or improving ASK1-related diseases, wherein the functional health food comprises a compound represented by Chemical Formula 1, or a stereoisomer, a solvate, a hydrate, or a pharmaceutically acceptable salt thereof.

The compound represented by Chemical Formula 1 according to the present invention exhibits high ASK1 inhibitory activity and may be prepared as health functional food compositions for preventing or treating ASK1-related diseases as a pharmaceutical composition standard, or may be added to functional health supplements such as foods and beverages.

The compound represented by Chemical Formula 1 of the present invention may be added to food as it is or used in combination with other foods or food ingredients, and may be used appropriately in accordance with conventional methods. The amount of the active ingredient can be determined appropriately according to the purpose of its use (for prevention or improvement). In general, the amount of the above compound in health food can be applied to 0.1 to 90 by weight of the total food weight. However, in the case of long-term consumption for health and hygiene purposes or for health control purposes, the above amount may be below the above range, and since there is no problem in terms of safety, the active ingredient can also be used in amounts higher the above range.

One embodiment of the present invention provides a method for preventing or treating ASK1-related diseases, comprising administering, to a subject, a pharmaceutical composition containing a compound represented by Chemical

Formula 1, or a stereoisomer, a solvate, a hydrate, or a pharmaceutically acceptable salt thereof as an active ingredient.

The above administration can be oral or parenteral. When administered orally, it can be administered in an amount of 0.01 to 1000 mg per kg of body weight per day, or more specifically, 0.1 to 300 mg per day based on the active ingredient, and parenterally administered in 1 to several doses to be administered at an amount of 0.01 to 100 mg per kg of body weight per day, or more specifically, 0.1 to 50 mg per day based on the active ingredient. The dose administered to a particular individual or patient should be determined in light of various relevant factors such as the patient's weight, age, gender, health status, diet, administration time, administration method, and severity of the disease, and may be appropriately added or subtracted by a specialist.

In this Specification, the term "individual" means an object requiring treatment of a disease and, more specifically, mammals such as primates, mice, dogs, cats, horses, and cattle, which are human or non-human.

One embodiment of the present invention provides the use of a compound represented by Chemical Formula 1, or a stereoisomer, a solvate, a hydrate, or a pharmaceutically acceptable salt thereof, for preventing or treating ASK1-related diseases.

One embodiment of the present invention provides the use of a compound, a stereoisomer, a solvate, a hydrate, or a pharmaceutically acceptable salt thereof, for the preparation of a drug for preventing or treating ASK1-related diseases.

Hereinafter, the present invention is described in details by examples and experimental examples.

However, the following embodiments and experimental examples are only examples of the present invention, and the contents of the present invention are not limited to the following embodiments and experimental examples.

**Manufacturing Example 1. 6-(1H-benzo[*d*][1,2,3]triazole-1-yl)pyridine-2-amine (Intermediate 1)**

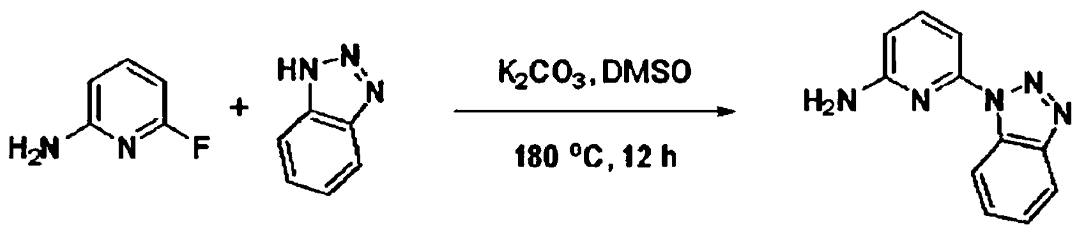


According to the above reaction scheme, Intermediate 1 was obtained.

A mixture of 6-fluoropyridine-2-amines (200 mg, 1.78 mmol), benzotriazole (255 mg, 2.14 mmol), and $K_2CO_3$ (740 mg, 5.34 mmol) in DMSO (6 mL) was heated at 180 °C for 12 hours. After cooling to room temperature, 1 M $Na_2CO_3$ solution was added. The resulting solids were filtered and dried under reduced pressure to obtain 6-(1H-benzo[d][1,2,3]triazole-1-yl)pyridine-2-amine (Intermediate 1, 230 mg, 61%). $^1$H NMR (500 MHz, Chloroform-d) δ 8.45 (d, *J* = 8.4 Hz, 1H), 8.11 (d, *J* = 8.3 Hz, 1H), 7.72 (t, *J* = 8.0 Hz, 1H), 7.59 (t, *J* = 7.7 Hz, 1H), 7.48 (d, *J* = 7.7 Hz, 1H), 7.44 (t, *J* = 7.7 Hz, 1H), 6.68 (d, *J* = 8.2 Hz, 1H); LCMS: 212.4 [M+H$^+$].

**Manufacturing Example 2. 6-(5,6-dihydrocyclopenta[d][1,2,3]triazole-1(4H)-yl)pyridine-2-amine (Intermediate 2)**

According to the following reaction scheme, Intermediate 2 was obtained.

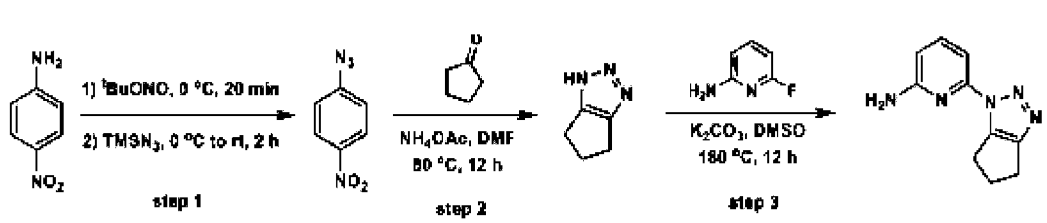


**Step 1: 1-Azido-4-nitrobenzene**

4-Nitroaniline (1.00 g, 7.24 mmol) was dissolved in anhydrous acetonitrile (20 mL) and then cooled to 0 °C in an ice bath. To the stirring solution, tert-butyl nitrite (1.03 mL, 8.69 mmol) was added, and the resulting mixture was stirred for 20 minutes. Then, azidotrimethylsilane (1.44 mL, 10.86 mmol) was added for 10 minutes, drop by drop, and the brown final

solution was stirred at room temperature for 12 hours. The solvent was removed under reduced pressure. The residue was purified using flash column chromatography (hexane/ethyl acetate 100/0 to 80/20) using silica gel to obtain 1-azidio-4-nitrobenzene (1.16 g, 97%).

$^1$H NMR (400 MHz, Methanol-*d*$_4$) δ 8.30 - 8.25 (m, 2H), 7.30 - 7.25 (m, 2H); LCMS: 165.5 [M+H$^+$].

**Step 2: 1,4,5,6-tetrahydrocyclopenta[d][1,2,3]triazole**

Cyclopentanone (1.08 mL, 12.2 mmol), $NH_4OAc$ (4.69 g, 60.9 mmol), and 1-azidio-4-nitrobenzene were added to a screw-capped reaction tube equipped with a magnetic stirring bar. The resulting mixture was dissolved in DMF (20 mL) and stirred at 80 °C for 12 hours. At completion, DMF was removed under reduced pressure and purified using flash column chromatography with silica gel (DCM 100%, hexane/ethyl acetate 100/0 to 50/50) to obtain 1,4,5,6-tetrahydrocyclopenta[*d*][1,2,3]triazole (164 mg, 61%).

$^1$H NMR (300 MHz, Chloroform-*d*) δ 10.03 (s, 1H), 2.88 - 2.78 (m, 4H), 2.66 - 2.54 (m, 2H).

**Step 3: 6-(5,6-dihydrocyclopenta[ d ][1,2,3]triazole-1(4 H)-yl)pyridine-2-amine**

A mixture of 6-fluoropyridine-2-amines (940 mg, 8.32 mmol), 1,4,5,6-tetrahydrocyclopenta[*d*][1,2,3]triazole (1.00 g, 9.16 mmol), and $K_2CO_3$ (3.44 g, 25.0 mmol) in DMSO (20 mL) was heated at 160 °C for 12 hours. After cooling to room temperature, the mixture was diluted with water and extracted twice with acetyl acetate. The combined organic layers was washed with brine, dried with $MgSO_4$, filtered, and concentrated. The residue was purified using flash column chromatography (hexane/ethyl acetate 100/0 to 60/40) using silica gel to obtain 6-(5,6-dihydrocyclopenta[d][1,2,3] triazole-1(4 h)-yl)pyridine-2-amine (Intermediate 2, 700 mg, 42%).

$^1$H NMR (300 MHz, Chloroform-*d*) δ 7.59 (t, J = 7.9 Hz, 1H), 7.41 (dd, J = 7.8, 0.7 Hz, 1H), 6.45 (dd, J = 8.0, 0.7 Hz, 1H), 3.18 - 3.05 (m, 2H), 2.88 - 2.76 (m, 2H), 2.73 - 2.59 (m, 2H); LCMS: 202.6 [M+H +].

**Manufacturing Example 3. 6-(4,5,6,7-tetrahydro-1 H-benzo[d][1,2,3]triazole-1-yl)pyridine-2-amine (Intermediate 3)**

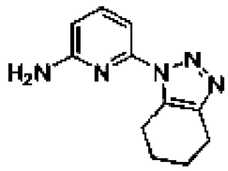

The same method was performed in Manufacturing Example 2 to obtain 6-(4,5,6,7-tetrahydro-1H-benzo[*d*] [1,2,3]triazole-1-yl)pyridine-2-amine (Intermediate 3, 39%).

$^1$H NMR (300 MHz, Chloroform-*d*) δ 7.60 (t, J = 7.9 Hz, 1H), 7.35 (dd, J = 7.8, 0.7 Hz, 1H), 6.48 (dd, J = 8.2, 0.7 Hz, 1H), 3.13 - 3.03 (m, 2H), 2.86 - 2.75 (m, 2H), 1.90 - 1.78 (m, 4H); LCMS: 216.6 [M+H$^+$].

**Manufacturing Example 4. 6-(5,6,7,8-tetrahydrocyclohepta[*d*][1,2,3]triazole-1(4 H)-yl)pyridine-2-amine (Intermediate 4)**

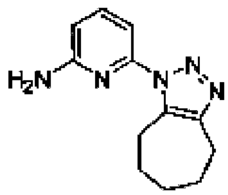

The same method was performed as in Manufacturing Example 2 to obtain 6-(5,6,7,8-tetrahydrocyclohepta[d] [1,2,3]triazole-1(4 H)-yl)pyridine-2-amines (Intermediate 4, 22%).

$^1$H NMR (400 MHz, Methanol-*d*$_4$) δ 7.65 (t, J = 7.9 Hz, 1H), 6.84 (d, J = 7.5 Hz, 1H), 6.67 (d, J = 8.3 Hz, 1H), 3.07 - 2.97 (m, 2H), 2.95 - 2.86 (m, 2H), 1.96 - 1.85 (m, 2H), 1.81 - 1.69 (m, 4H); LCMS: 230.6 [M+H+].

**Manufacturing Example 5. 6-(4,5,6,7,8,9-hexahydro-1H-cycloocta[*d*][1,2,3]triazole-1-yl)pyridine-2-amine (Intermediate 5)**

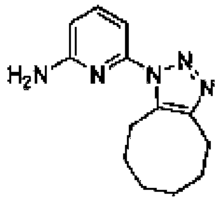

The same method was performed in Manufacturing Example 2 to obtain 6-(4,5,6,7,8,9-hexahydro-1H-cycloocta [*d*][1,2,3]triazole-1-yl)pyridine-2-amines (Intermediate 5, 23%).

$^1H$ NMR (500 MHz, Chloroform-*d*) δ 7.65 (t, *J* = 7.9 Hz, 1H), 7.20 - 7.10 (m, 1H), 6.74 - 6.63 (m, 1H), 3.17 - 3.09 (m, 2H), 3.06 - 2.99 (m, 2H), 1.94 - 1.87 (m, 2H), 1.85 - 1.76 (m, 2H), 1.57 - 1.45 (m, 4H); LCMS: 244.6 [$M+H^+$].

**Manufacturing Example 6. 6-(4,5,6,7-tetrahydro-1H-benzo[*d*][1,2,3]triazole-1-yl)pyridine-2-amines (an alternative method of preparation of Intermediate 3)**

The Intermediate 3 can also be prepared according to the following reaction scheme.

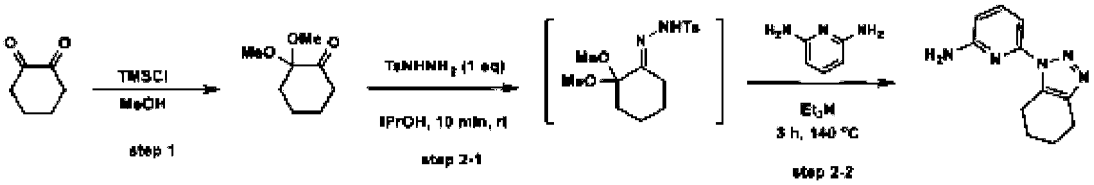


**Step 1. 2,2-Dimethoxycyclohexane-1-one**

A solution of cyclohexyl-1,2-dione (6.0 g, 53.5 mmol) in methanol (50 ml) was slowly added to a solution of trimethylsilyl chloride (7.0 ml, 58.9 mmol) in methanol (500 ml), and the resulting mixture was stirred at room temperature for 16 hours. The reaction mixture was poured into water and extracted with diethyl ether. The organic layer was washed with saturated ammonium carbonate solution, water, and salt water. After drying with $MgSO_4$, the solvent was removed under reduced pressure to obtain 2,2-dimethoxycyclohexane-1-one (quantitative yield).

$^1H$ NMR (400 MHz, DMSO-$d_6$) δ 3.14 (s, 6H), 2.39 (t, *J* = 6.6 Hz, 2H), 1.85 (t, *J* = 6.0 Hz, 2H), 1.77 - 1.68 (m, 2H), 1.68 - 1.60 (m, 2H).

**Step 2. 6-(4,5,6,7-tetrahydro-1H-benzo[*d*][1,2,3]triazole-1-yl)pyridine-2-amines**

4-Methylbenzenesulfonohydrazide (6.0 g, 32.2 mmol) was added to a solution of 2,2-dimethoxycyclohexane-1-one (5.6 g, 35.4 mmol) in isopropanol (100 ml) and stirred at room temperature for 30 min (at this stage, consumption of 4-methylbenzenesulfonohydrazide was observed). Pyridine-2,6-diamine (3.86 g, 35.4 mmol) and triethylamine (4.9 ml, 35.4 mmol) were added to the mixture. After 3 hours at 140°C, the mixture was cooled to room temperature, and the reaction mixture was divided between dichloromethane and water, and then the organic layer was separated. The water layer was extracted two more times with dichloromethane, and the combined organic layers was washed with brine, dried over $MgSO_4$ and concentrated. The product was purified using flash column chromatography (hexane/ethyl acetate 100/0 to 50/50) using silica gel to obtain 6-(4,5,6,7-tetrahydro-1H-benzo[*d*][1,2,3]triazole-1-yl)pyridine-2-amines (Intermediates 3, 3.0 g, 43%).

$^1H$ NMR (400 MHz, DMSO-$d_6$) δ 7.59 (t, *J* = 7.9 Hz, 1 H), 6.99 (d, *J* = 7.5 Hz, 1 H), 6.48 (d, *J* = 8.2 Hz, 1 H), 6.33 (s, 2 H), 3.03 (d, *J* = 3.2 Hz, 2 H), 2.68 (s, 2 H), 1.84 - 1.72 (m, 4 H); LCMS [M+H]; 216.27.

**Example 1. N-(6-(1H-benzo[d][1,2,3]triazole-1-yl)pyridine-2-day)-5-(4-cyclopropyl-1H-imidazole-1-day)-2-fluoro-4-methylbenzamide**

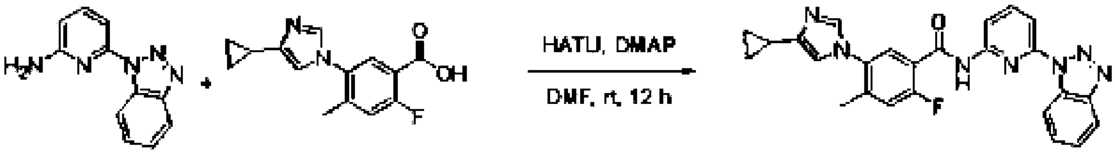


Example 1 compound was prepared according to the reaction formula.

HATU (1.25 g, 3.30 mmol) was added to a stirred solution of 6-(1-H-benzo[d][1,2,3]triazole-1-yl)pyridine-2-amine (Intermediate 1, 70 mg, 0.33 mmol), DMAP (400 mg, 3.30 mmol), and 5-(4-cyclopropyl-1H-imidazole-1-yl)-2-fluoro-4-methylbenzoic acid (130 mg, 0.50 mmol) [purchased from AA blocks (5 g), Labnetwork (10 g), Chemscene; reference: WO 2013/112741] in DMF (3 mL) with stirring. The resulting mixture was stirred at room temperature for 12 hours, then diluted with ethyl acetate and saturated aqueous $NaHCO_3$ solution. The separated organic layer was washed with brine, dried with anhydrous $MgSO_4$, and filtered. The organic filtrate was evaporated under reduced pressure. The obtained material was purified using flash column chromatography using silica gel (ethyl acetate/methanol 100/0 to 90/10) and C18-reversed-phase HPLC (using % TFA in H2O and 0.1% TFA in ACN as buffer solvents), and N-(6-(1 H -benzo[d][1,2,3] triazole-1-yl)pyridine-2-yl)-5-(4-cyclopropyl-1H-imidazole-1-yl)-2-fluoro-4-methylbenzamide (1, 35 mg, 23%).

$^{1}H$ NMR (300 MHz, Methanol-$d_4$) δ 9.07 (d, *J* = 1.7 Hz, 1H), 8.84 (dd, *J* = 8.4, 1.0 Hz, 1H), 8.29 (dd, *J* = 7.6, 1.3 Hz, 1H), 8.18 - 8.04 (m, 3H), 7.97 (d, *J* = 6.4 Hz, 1H), 7.70 - 7.63 (m, 1H), 7.60 - 7.56 (m, 1H), 7.56 - 7.50 (m, 1H), 7.47 (d, *J* = 10.8 Hz, 1H), 2.34 (s, 3H), 2.12 - 2.01 (m, 1H), 1.19 - 1.10 (m, 2H), 0.91 (dt, *J* = 7.0, 4.7 Hz, 2H); LCMS: 454.6 [M+H$^+$].

**Example 2. 5-(4-cyclopropyl-1H-imidazole-1-day)-N-(6-(5,6-dihydrocyclopenta[*d*][1,2,3]triazole-1(4 h)-day)pyridine-2-day)-2-fluoro-4-methylbenzamide**

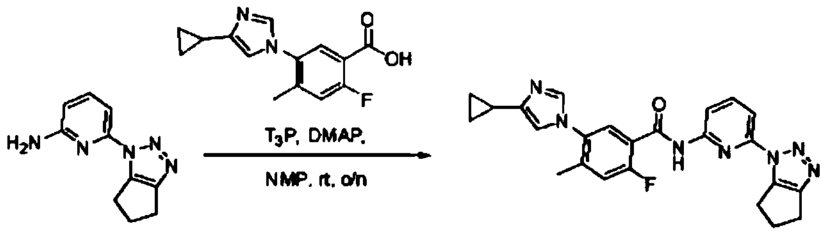


DMAP (0.36 g, 3.0 mmol) was added to a stirred solution of 5-(4-cyclopropyl-1H-imidazole-1-yl)-2-fluoro-4-methylbenzoic acid (0.38 g, 1.3 mmol) in NMP (3.0 ml), followed by slow addition of T3P (50% ethyl acetate) (1.27 ml, 2.0 mmol) at room temperature. The resulting mixture was stirred for 10 minutes 6-(5,6-dihydrocyclopenta[d][1,2,3]triazole-1(4H)-yl)pyridine-2-amines (0.2 g, 1.0 mmol) was added, and the mixture was stirred for 12 hours. The reaction mixture was quenched with ice cold water and basified (pH 7-8) with saturated aqueous $NaHCO_3$ solution. The resulting solids were filtered, washed with water and dried. The desired compound 5-(4-cyclopropyl-1H-imidazole-1-yl)-N-(6-(5,6-dihydrocyclopenta[*d*][1,2,3]triazole-1(4H)-yl)pyridine-2-yl)-2-fluoro-4-methylbenzamide was obtained by osmosis of the solid with ethyl acetate as a pure white solid (0.3 g, 68%).

$^{1}H$ NMR (400 MHz, DMSO) δ 10.96 (s, 1 H), 8.19 - 8.08 (m, 2 H), 7.78 (dd, *J* = 7.6, 1.0 Hz, 1 H), 7.69 (d, *J* = 1.4 Hz, 1 H), 7.63 (d, *J* = 6.6 Hz, 1 H), 7.48 (d, *J* = 10.8 Hz, 1 H), 7.18 (d, *J* = 1.4 Hz, 1 H), 3.14 (t, *J* = 7.1 Hz, 2 H), 2.78 - 2.70 (m, 2 H), 2.67 - 2.57 (m, 2 H), 2.25 (s, 3 H), 1.85 (td, J = 8.3, 4.1 Hz, 1 H), 0.83 - 0.77 (m, 2 H), 0.73 - 0.67 (m, 2 H): LCMS; [M+H]$^+$ 444.6.

**Example 3. 5-(4-cyclopropyl-1H-imidazole-1-yl)-2-fluoro-4-methyl-N-(6-(4,5,6,7-tetrahydro-1 H-benzo[*d*][1,2,3] triazole-1-yl)pyridine-2-yl)methylbenzamide**

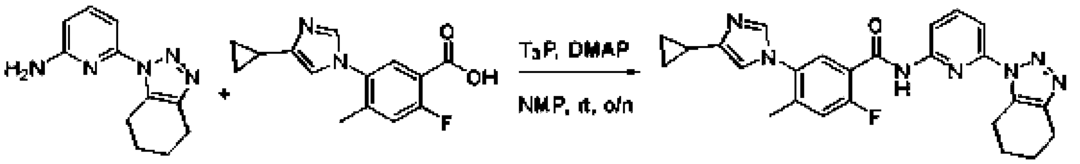

DMAP (4.25 g, 34.8 mmol) was applied to a stirred solution of commercially available 5-(4-cyclopropyl-1H-imidazole-1-yl)-2-fluoro-4-methylbenzoic acid (3.93 g, 15.1 mmol) in NMP (30 ml), followed by slow addition of T3P (50% ethyl acetate) (14.7 g, 23.2 mmol) at room temperature. The resulting mixture was stirred for 10 minutes, and 6-(4,5,6,7-tetrahydro-1H-benzo[d][1,2,3]triazole-1-yl)pyridine-2-amines (2.5 g, 11.6 mmol) was added and stirred for 12 hours. The reaction mixture was quenched with ice cold water and basified (pH 7-8) with saturated aqueous $NaHCO_3$ solution. The resulting solids were filtered, washed with water and dried. The desired 5-(4-cyclopropyl-1H-imidazole-1-yl)-2-fluoro-4-methyl-N-(6-(4,5,6,7-tetrahydro-1H-benzo[d][1,2,3]triazole-1-yl)pyridine-2-yl)benzamide compound was obtained as a white solid (5.0 g, 94%) by osmosing the solid with methanol.

$^1H$ NMR (400 MHz, DMSO) δ 11.03 (s, 1 H), 8.19 (dd, *J* = 8.3, 0.9 Hz, 1 H), 8.12 (t, *J* = 8.0 Hz, 1 H), 7.75 (dd, *J* = 7.8, 0.8 Hz, 1 H), 7.70 (d, *J* = 1.5 Hz, 1 H), 7.63 (d, *J* = 6.5 Hz, 1 H), 7.48 (d, *J* = 10.7 Hz, 1 H), 7.18 (d, *J* = 1.4 Hz, 1 H), 3.11 (d, *J* = 5.3 Hz, 2 H), 2.70 (d, *J* = 4.6 Hz, 2 H), 2.25 (s, 3 H), 1.90 - 1.82 (m, 1 H), 1.81 - 1.75 (m, 4 H), 0.84 - 0.78 (m, 2 H), 0.73 - 0.67 (m, 2 H). LCMS [M+H]; 458.57.

**Example 4. 5-(4-cyclopropyl-1H-imidazole-1-yl)-2-fluoro-4-methyl-N-(6-(5,6,7,8-tetrahydrocyclohepta[*d*][1,2,3]triazole-1(4H)-yl)pyridine-2-yl)benzamide**

The same method was performed as in Example 1, except that Intermediate 4 was used instead of Intermediate 1, and 5-(4-cyclopropyl-1H-imidazole-1-yl)-2-fluoro-4-methyl-N-(6-(5,6,7,8-tetrahydrocyclohepta[d][1,2,3]triazole-1(4H)-yl)pyridine-2-yl)benzamide was obtained (13%).

$^1H$ NMR (400 MHz, Methanol-*$d_4$*) δ 9.03 (d, *J* = 1.6 Hz, 1H), 8.39 (d, *J* = 8.3 Hz, 1H), 8.11 (t, *J = 8.1* Hz, 1H), 7.94 (d, J = 6.4 Hz, 1H), 7.60 (d, *J* = 7.8 Hz, 1H), 7.57 - 7.52 (m, 1H), 7.46 (d, *J* = 10.9 Hz, 1H), 3.18 - 3.12 (m, 2H), 2.94 - 2.89 (m, 2H), 2.32 (s, 3H), 2.08 - 2.01 (m, 1H), 1.96 - 1.88 (m, 2H), 1.82 - 1.73 (m, 4H), 1.16 - 1.10 (m, 2H), 0.92 - 0.87 (m, 2H); LCMS: 472.5 [$M+H^+$].

**Example 5. 5-(4-cyclopropyl-1H-imidazole-1-yl)-2-fluoro-N-(6-(4,5,6,7,8,9-hexahydro-1 H-cycloocta[d][1,2,3]triazole-1-yl)pyridine-2-yl)-4-methylbenzamide**

The same procedure was performed as in Example 1 to obtain 5-(4-cyclopropyl-1H-imidazole-1-yl)-2-fluoro-N-(6-(4,5,6,7,8,9-hexahydro-1H-cycloocta[d][1,2,3]triazole-1-yl)pyridine-2-yl)-4-methylbenzamide (4%).

$^1H$ NMR (500 MHz, Methanol-*$d_4$*) δ 9.10 (d, *J* = 1.7 Hz, 1H), 8.38 (d, *J* = 8.2 Hz, 1H), 8.11 (t, *J* = 8.1 Hz, 1H), 7.99 (d, *J* = 6.4 Hz, 1H), 7.70 (d, *J* = 7.8 Hz, 1H), 7.58 (d, *J* = 1.3 Hz, 1H), 7.48 (d, *J* = 11.0 Hz, 1H), 3.25 - 3.20 (m, 2H), 2.97 - 2.92 (m, 2H), 2.33 (s, 3H), 2.08 - 2.02 (m, 1H), 1.96 - 1.89 (m, 2H), 1.82 - 1.75 (m, 2H), 1.59 - 1.54 (m, 2H), 1.54 - 1.48 (m, 2H), 1.18 - 1.11 (m, 2H), 0.94 - 0.88 (m, 2H); LCMS: 486.5 [$M+H^+$].

The structural formulas of the compounds prepared in Examples 1 to 5 are summarized in Table 1 below.

[Table 1]

| Example | Chemical Structure | Example | Chemical Structure |
|---|---|---|---|
| 1 | 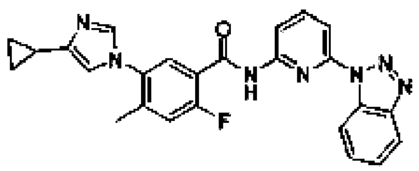 | 2 | 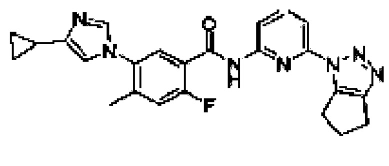 |
| 3 | 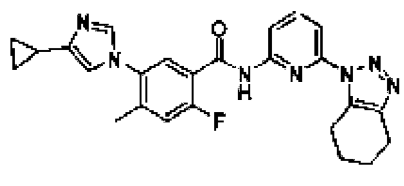 | 4 | 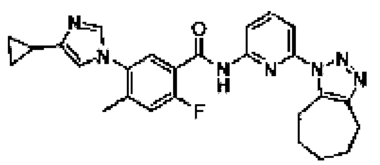 |
| 5 | 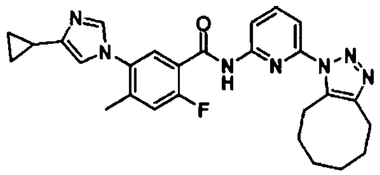 | | |

**Experimental Example 1. Evaluation of ASK1 Inhibition Activity**

The following experiments were performed to measure the inhibitory ability of ASK1 activity of a compound represented by Chemical Formula 1 according to the present invention.

The recombinant human ASK1 protein was initiated by adding a mixture of Mg/ATP to the reaction solution (8 mM MOPS pH 7.0, 0.2 mM EDTA, 0.33 mg/mL myelin basic protein, 10 mM Mg Acetate and [gamma-33P]-ATP), and the reaction was terminated by adding 0.5% phosphoric acid after 40 min at room temperature. After that, 10 μL of the reaction solution was dropped into the P30 drip filter, washed in 0.425% phosphoric acid for 4 minutes a total of 4 times, added into methanol to dry, and the activity of ASK1 was measured by scintillation counting. The activity value from the group that did not contain the compound was set at 100% to convert the degree of ASK2 inhibition by the compound, and the ASK1 inhibition activity of the Example compounds was summarized in Table 2 below.

[Table 2]

| Example | Remaining kinase activity (%) at 0.5 uM | $IC_{50}$ (nM) |
|---|---|---|
| 1 | 14 | 235 |
| 2 | 2 | 37 |
| 3 | 7 | 73 |
| 4 | - | 92 |
| 5 | - | 198 |
| Comparative Example 1 (Selonsertib) | 0 | 107 |

Looking at Table 2 above, it was confirmed that the compound represented by Chemical Formula 1 according to the present invention is a substance with ASK1 inhibitory activity. Therefore, the compound of the present invention is useful as a composition for preventing or treating neurodegenerative brain diseases caused by ASK1.

**Experimental Example 2. Evaluation of Cytoprotective Effects in MPP+-Induced Human Neurons**

The following experiments were performed to evaluate the effect of protecting the damage of dopaminergic neurons by a compound represented by Chemical Formula 1 according to the present invention.

SH-SY5Y cells were purchased from ATCC and cultured at 37°C and 5% $CO_2$ in RPMI medium containing 10% FBS and 1% penicillin and streptomycin. MPP+ was purchased from Sigma Aldrich and treated with 5 mM to induce cytotoxicity. Cell viability was measured at 540 nm after treatment with CCK-8 reagent for 1-3 hours, and the results are shown in Table 3 below.

[Table 3]

| Example | $EC_{50}$ (nM) in SH-SY5Y |
|---|---|
| 2 | 64 |
| 3 | 23 |
| Comparative Example 1 (Selonsertib) | 89 |

As shown in above Table 3 and FIG. 1, the Example compounds of the present invention in SH-SY5Y cells exhibited excellent cytoprotective effects, especially embodiments 2 and 3 showed superior $EC_{50}$ values over Comparative Example 1 (Selonsertib).

**Experimental Example 3. Assess cell viability**

To assess cell viability during compound treatment, BV2 cells were seeded in 96-well plates with four pieces of 2x10 per well and incubated for one day, then treated with 0.1 μM, 0.3 μM, and 1 μM, respectively, and incubated at 5% $CO_2$ at 37°C for 72 hours. Cell viability was measured by adding the Cell Counting Kit-8 (CCK-8; LPS solution, Seoul, Korea) to the cultured cells, and quantified using absorbance measured at 565 nm wavelength using a Hidex sense microplate reader (Hidex, Turku, Finland).

As shown in FIG. 2, the Example compound according to the present invention exhibited a similar level of cell

viability to the Comparative Example 1 (Selonsertib).

**Experimental Example 4. Evaluation of anti-inflammatory efficacy**

BV2 cells (2 x 10 4 cells per well) were incubated for 24 hours in a 96-well culture plate. Subsequently, the Example compounds were added in 0.1 μM, 0.3 μM, and 1 μM, respectively, for 1 hour, followed by the addition of LPS 100 pg/ml and incubated for 24 hours. After incubation, conditioned medium (CM) was harvested and centrifuged at 2000 rpm. After separating the supernatant of the solution after centrifugation, the TNF-α secreted from the cell culture medium was measured using the corresponding ELISA kit (LABISKOMA, Seoul, Korea).

As shown in FIG. 2, the Example compounds according to the present invention reduced the secretion of the inflammatory cytokine TNF-α induced by LPS to a level similar to that of Selonsertib (Comparison Example 1). In particular, it was confirmed that Example 3 compound reduced TNF-α secretion at all concentrations compared to Selonsertib, confirming that it has excellent anti-inflammatory efficacy.

**Experimental Example 5. Evaluation of neuronal protective efficacy**

The neuronal toxicity substances 6-OHDA (6-hydroxydopamine hydrobromide) and MPP$^+$ (1-methyl-4-phenylpyridinium) were purchased from Sigma-Aldrich (Saint Louis, MO, USA) and made into 100 mM DMSO stock. PC12 cells were seeded on 96-well plates with 4 pieces of 1x10 per well and incubated for one day. The cells were pretreated with each concentration of embodied compound for 1 h. Subsequently, 40 μM 6-OHDA and 2 mM MPP$^+$ were added and incubated for 24 hours, respectively. Subsequently, cell viability was measured using MTT (Invitrogen, MA, USA).

As shown in Tables 4 and 5, the embodiment compound according to the present invention showed superior cell viability than the comparative example 1 (selonsertib), and thus confirmed that it has an excellent neuronal protective effect in the PC-12 cell line.

[Table 4]

| Group | Control | 6-OHDA | Comparative Example 1 (μM) (Selonsertib) | | | Example 3 (μM) | | | Example 2 (μM) | | |
|---|---|---|---|---|---|---|---|---|---|---|---|
| | | (40 μM) | 0.1 | 0.3 | 1 | 0.1 | 0.3 | 1 | 0.1 | 0.3 | 1 |
| AVE | 100 | 81.51 | 87.91 | 89.18 | 84.09 | 89.95 | 91.98 | 92.77 | 88.85 | 91.49 | 96.78 |
| STDEV | 0.9 | 1.83 | 0.92 | 1.12 | 2.31 | 1.37 | 1.02 | 2.85 | 2.33 | 1.42 | 1.28 |

[Table 5]

| Group | | Control | | MPP | | Comparison Example 1 (μM) (Selonsertib) | | Example 3 (μM) | | Example 2 (μM) | |
|---|---|---|---|---|---|---|---|---|---|---|---|
| | | (2 mM) | 0.1 | 0.3 | 1 | 0.1 | 0.3 | 1 | 0.1 | 0.3 | 1 |
| AVE | 100 | 80.09 | 83.58 | 81.31 | 81.31 | 87.4 | 87.56 | 89.89 | 83.87 | 91.71 | 92.44 |
| STDEV | 1.74 | 2.37 | 2.39 | 1.94 | 1.40 | 3.72 | 1.02 | 1.55 | 2.98 | 0.23 | 2.07 |

**Experimental Example 6. Evaluate the effect of TG synthesis inhibition**

Triglyceride (TG) measurement was performed by aliquoting HepG2 cells in human liver cancer cells on 96-well plates with 4 cells of 2x10 per well for 24 hours. Cultured cells were pretreated with the Example compound by concentration for 2 hours, followed by the addition of 1 mM of free fatty acid (FFA) mixed with palmitic acid (PA, sigma, #P9767) and oleic acid (OA, sigma, #O7501) in a 1:2 ratio, and incubated for 48 hours. After incubation, the supernatant was isolated and measured at absorbance at 570 nm using the Triglyceride Quantification Kit (Sigma, #MAK266).

As shown in Table 6 below, the anti-NASH (non-alcoholic steatohepatitis) efficacy was verified using TG synthesis inhibition. As a result of the experiment, the compound of Example 3 showed a better inhibition effect on TG synthesis with increasing treatment concentrations.

[Table 6]

| | HepG2 | FFA 1mM + Example 3 (0 μM) | FFA 1mM + Example 3 (0.3 μM) | FFA 1mM + Example 3 (1 μM) |
|---|---|---|---|---|
| Cell Viability (% of HepG2 control) | 100 | 93.97 | 99.06 | 93.10 |
| TG Level (% of HepG2 control) | 100 | 125.86 | 117.38 * | 106.59 * |

**Experimental Example 7. MDR-MDCK cell membrane permeability assessment**

MDCK-MDR1 cells (5x10 5cells/mL) were incubated on a 24-transwell plate (Corning, NY, USA) for 4 days to measure permeability through a single layer of MDR-MDCK cells. The medium was replaced with a new medium every two days, and the electrical resistance between epithelial tissues (TEER) was measured using a Millicell® ERS-2 Volohmmeter (Merck KGaA, Darmstadt, Germany), and the cell membrane permeability test was performed after confirming that this value was greater than or equal to 300 Ohm.cm$^2$.

The apical and basolateral chambers were washed twice each in the order of Hanks' Balanced Salt Solution (HBSS) containing DPBS buffer and 10 mM Hydroxyethyl piperazine Ethane sulfonic acid (HEPES), and in the final washing step, HBSS was filled and maintained at a stable state at 37°C for 30 minutes. After 30 minutes, the test material was instilled in each chamber and left at 37°C for 2 hours, and the culture medium of the transferred layer was collected at 30-minute intervals, and the collected amount was supplemented with new HBSS.

The apparent permeability coefficient (Papp, cm/sec) was calculated using the following formula:

[Mathematical Equation 1]

Papp =dQ/dt X 1/A X 1/C_0

* Papp : 겉보기 투과 계수 (Apparent permeability)
* dQ/dt : Permeability of the drug
* A: Area of the filter membrane on which the cells are laid
* C_0: Initial concentration of the drug

In addition, the Efflux ratio was calculated as follows:

[Mathematical Equation 2]

Efflux ratio (ER) = (Papp (Basolateral to Apical))/(Papp (Apical to Basolateral))

[Table 7]

| Example | Papp (x $10^{-6}$ cm/sec) | | Efflux ratio |
|---|---|---|---|
| | A to B | B to A | |
| 2 | 93.2 ± 11.2 | 99.1 ± 16.8 | 1.06 |
| 3 | 67.3 ± 12.8 | 74.8 ± 12.3 | 1.13 |
| 4 | 57.3 ± 4.23 | 87.4 ± 3.39 | 1.53 |
| Comparative Example1 (Selonsertib) | 3.82 ± 0.66 | 102 ± 2.97 | 27 |

As shown in Table 7, the Comparative Example 1 compound (Selonsertib) shows an efflux coefficient of 27 due to the large difference in the apparent coefficient of the absorption direction and the outflow direction, and it is confirmed that it is a substrate of the effluent transporter, while the embodied compounds according to the present invention exhibit a very high apparent multiple value and an outflow ratio close to 1 in both the absorption and outflow directions, making it easy for

cell membrane permeability and not a substrate of the effluent transporter.

**Experimental Example 8. Distribution in the mouse brain**

The blood-brain barrier permeability in ICR mice was confirmed by oral administration of 10 mg/kg of test material. Male C57BL/6 mice were fasted for 15 h prior to administration. The test material was administered at a dose of 10 mg/kg and a volume of 5 mL/kg, and the solvent composition was N-Methyl-2-pyrrolidone (NMP), Polyethylene glycol 400 (PEG 400), and distilled water (D.W) at a volume ratio of 10:40:50 (%), respectively. After administration, blood and brain tissue samples were taken by sacrificing mice with carbon dioxide inhalation at each time point corresponding to 1, 4, and 8 hours. Whole blood was collected from the abdominal vena cava, and brain samples were collected after systemic perfusion with saline to remove as much blood as possible.

The distribution ratio in mouse plasma and brain was calculated by calculating the AUC after concentration quantification in each tissue.

[Mathematical Equation 3]

$$Kp = (\text{Total brain AUC})/(\text{Total plasma AUC})$$

[Table 8]

| Example | $Auk_{inf}$ (ug*h.ml or ug*h.g.) | | Kp |
|---|---|---|---|
| | plasma | brain | |
| Example 3 | 320 ± 74.8 | 6.61 ± 1.75 | 0.021 |
| Comparative Example1 (Selonsertib) | 69.9 ± 3.48 | 0.26 ± 0.03 | 0.004 |

As shown in Table 8, it is confirmed that the Example 3 according to the present invention has relatively very high concentration and Kp values in plasma and brain compared to Comparison Example 1 (Selonsertib), and therefore has excellent pharmacokinetic properties for the treatment of brain diseases.

**Experimental Example 9. Evaluation of Efficacy in MPTP-Induced Parkinson's Disease Mouse Model**

**Experimental Example 9.1. Preparing for the experiment**

All animal experiments described in this specification were approved by the Ethics Committee (IACUC) and performed in accordance with the Ministry of Food and Drug Safety's guidelines on the management and use of laboratory animals.

A C57BL/6 male mouse was purchased from Koatech (Korea) at 6 weeks of age. Mice were raised in groups of up to 4 per cage in a normal light and dark cycle (08:00-20:00) in an environment with controlled temperature (22±1°C) and humidity (30-50%), and food and water were provided with unlimited food and water (Ad libitum). The mice were allowed to adapt for one week before the start of the experiment.

The test drug was administered to normal mice and a mouse model of Parkinson's disease (PD) induced by MPTP (1-methyl-4-phenyl-1,2,3,6-tetrahydropyridine) and its efficacy was evaluated. For the compounds in the embodiment, the test drug was prepared using 10% NMP, 40% PEG 400, and 50% D.W. (distilled water) as the vehicle.

In the evaluation results, all values were expressed as mean ± standard error (SEM) and statistically analyzed by one-way ANOVA through LSD (Least Significant Difference) post-hoc analysis. The significance level of the evaluation results was set according to the following criteria.

Significance level compared to G1 - *: $p < 0.05$, **: $p < 0.01$, ***: $p < 0.001$
Significance level compared to G2 - #: $p < 0.05$, ##: $p < 0.01$, ###: $p < 0.001$

**Experimental Example 9.2. Design of experiments**

FIG. 3 is a schematic diagram of an experiment for an efficacy experiment on an MPTP-induced PD mouse

model.

Mice at 7 weeks of age were divided into 7 groups as shown in Table 9 below to generate an acute MPTP model and proceed with drug administration: control group + vehicle (G1), MPTP + vehicle (G2), MPTP + Nilotinib 40 mg/kg (G3), MPTP + Example 3 2 mg/kg (G4), MPTP + Example 3 10 mg/kg (G5), MPTP + Example 3 50 mg/kg (G6), and MPTP + Example 2 50 mg/kg (G7). The test drug was administered orally (PO) at 10 ml/kg once daily (QD) from Day 1 to Day 7. The acute MPTP model will be performed at 2-hour intervals in all groups except the control group. HCl (free base 20 mg/kg) was administered as intraperitoneal (IP) injection for 4 doses. The control group was given the same amount of 0.9% saline. The Pole test was performed on day 4 (after training on day 2 and day 3). On day 7, all mice were euthanized, and their brains were harvested.

During the study period, the weight was measured daily and the mice were checked for any abnormalities. If the general health of the mice deteriorated significantly, the mice were euthanized and removed from the study.

[Table 9]

| Group | Number of Animals | Type | Treatment Drug | Route of Administration | Dose |
|---|---|---|---|---|---|
| G1 | 12 | Saline solution | Carrier | PO, QD | - |
| G2 | 12 | MPTP | Carrier | PO, QD | - |
| G3 | 11 | MPTP | Nilotinib | PO, QD | 40 mg/kg |
| G4 | 12 | MPTP | Example 3 | PO, QD | 2 mg/kg |
| G5 | 12 | MPTP | Example 3 | PO, QD | 10 mg/kg |
| G6 | 12 | MPTP | Example 3 | PO, QD | 50 mg/kg |
| G7 | 11 | MPTP | Example 2 | PO, QD | 50 mg/kg |

**Experimental Example 9.3. Evaluation of behavioral deficit improvement efficacy**

A vertical wooden pole (0.8 cm to 1 cm in diameter and 80 cm in length, which provides grip for the mouse) was placed on the floor in a cage and tested. The mice were trained for two consecutive days (day 2 and day 3), and each training session consisted of three trials. On the third day (the actual test day, Day 4), the total time it took to reach the bottom of the pillar was measured by placing the mouse on the tip of the pillar and turning around the top, and the total time it took to reach the bottom was recorded separately from the top (turn down), climb down (the time it took to reach the floor from the time the head was lowered), and the total time it took to touch the floor (total time) was recorded, and the graph in FIG. 4 was obtained.

FIG. 4 is a graph of the results of the behavioral deficit assessment (pole test) for the MPTP-guided PD mouse model. In the pole test, it was confirmed that the group (G2) treated with MPTP took significantly more rotation (a in FIG. 4), descent (b in FIG. 4), and total time to reach the ground (c in FIG. 4) compared to the control group (G1). The group treated with MPTP and the embodiment compound (G4 to G7) had a significantly reduced time required to rotate compared to group G2. In particular, there was a significant reduction in rotation, descent, and ground reach time in groups G4 and G5. Through this, it was confirmed that the embodied compound significantly improved the behavioral defects.

**Experimental Example 9.4. Assessing the degree of TH staining**

Mice were euthanized with Zoletil administration for collection of tissue samples for staining and analysis of histological sections. Blood was collected from the heart of the mouse and then the residual blood was perfused with phosphate-buffered saline (PBS). For half of the mice (n=6/group), the brain was incised into the striatum, substantia nigra, and the rest of the brain, flash-frozen, and stored at -80 °C. The remaining mice (n=6/group) had their brains isolated and immobilized in 4% paraformaldehyde for 24 hours at 4 °C, followed by 30% sucrose in phosphate-buffered saline (PBS) containing 0.02% sodium azide and stored at 4 °C for 3 days. Immunohistochemical sections were performed on brain samples embedded with optimal cutting temperature (OCT) compounds. Samples were sectioned to a thickness of 20 μm using Leica CM1950 (Leica Microsystems, Germany) and stored at 4 °C in a preservation solution until staining with antibodies.

TH (tyrosine hydroxylase) is a catalytic enzyme that converts the amino acid L-tyrosine into L-3,4-dihydroxyphenylalanine (L-DOPA) and is one of the most important elements in the biosynthesis of catecholamines, including dopamine (DA). Changes in the expression or activity of TH have a significant impact on DA production and are therefore very important in the pathogenesis of Parkinson's disease.

After staining the striatum and substantia nigra of the brain and mounting them on microscopic slides, the slides

were washed with PBS containing 0.3% Triton X-100 and peroxidase block for 15 min at room temperature. The slides were washed three times with PBS containing 0.3% Triton X-100 and then blocked at RT (room temperature) for 1 hour in a solution containing PBS, 5% normal goat serum, and 0.3% Triton X-100. Tyrosine hydroxylase antibody was diluted at 1:1000 and incubated overnight at 4°C. The slides were washed with PBS solution and incubated for 1 h at RT with secondary antibody. The slides were washed with PBS solution and the sections were placed in the solution to proceed with the DAB (diaminobenzidine) reaction.

The DAB response was monitored under a microscope for 2 to 3 minutes, and the slides were washed with PBS to stop the DAB response, dehydrated, and mounted using xylene. An Olympus microscope (Olympus, SZ61, BX51) was used to visualize TH stained images. The optical density (OD) value of positive TH staining was measured in the striatum and substantia nigra and analyzed using Image J software.

FIG.s 5a and 5b are the results of TH staining immunohistochemistry analysis for striatum, and FIG.s 6a and 6b are results of TH staining immunohistochemistry analysis for substantia nigra. TH levels in the group treated with MPTP in the striatum and substantia nigra (G2) were significantly lower than in the control group (G1). The striatal TH levels in the nilotinib treated group (G3) were significantly higher than those in G2. The group treated with embodiment 3 or embodiment 2 had a significant increase in TH levels in both striatum and substantia nigra compared to G2. In addition, TH levels in the striatum at G5 (MPTP + 10 mg/kg embodiment3) and G6 (MPTP + 50 mg/kg embodiment3) and TH levels in the substantia nigra in G5, G6, and G7 (MPTP + 50 mg/kg embodiment2) were significantly higher than in the control group (G1).

## Experimental Example 9.6. Evaluating Dopamine-Increasing Efficacy

The amount of dopamine was measured by LC-MS/MS analysis using Agilent 1290 (LC) and SCIEX QTARP 6500 + (QQQ) for the striatum and substantia nigra of the extracted brain tissue.

FIG. 7 is a graph of dopamine levels by group in the striatum and substantia nigra of an MPTP-induced PD mouse model. Referring to FIG. 7, the dopamine concentration in both the striatum and the substantia nigra in the 50 mg/kg treatment group in Example 2 and in the substantia nigra group was significantly increased compared to the MPTP alone treatment group in the substantia nigra group, and in the substantia nigra in the 2 and 10 mg/kg treatment groups in Example 3 compared to the MPTP alone group. Through this, it was confirmed that the embodied compound significantly increased the amount of dopamine.

## Experimental example 10. Evaluation of Efficacy in α-Syn Transgenic Parkinson's Disease Mouse Model

Experimental Example 10.1. Preparing for the experiment

mThy-1α-syn Transformation (α-syn TG; Line No. 61) females obtained from University of California San Diego (UCSD) were crossed with C57Bl/6 X DBA/2 F1 WT (wild) males and bred at the Animal Center of the Osong Advanced Medical Industry Promotion Foundation (KBIOHealth).

All mice were raised in a normal light-dark cycle (08:00-20:00) in an environment with controlled temperature (22 ±1°C) and humidity (30-50%), and were provided with unlimited food and water (Ad libitum). Ear markers were made at the age of 15 to 21 days of age, ear or tail samples were taken, and polymerase chain reaction (PCR) analysis for genotyping was performed. In addition, tail samples were collected at the end point for genotyping if necessary.

The test drug was administered to normal mice and α-Syn (alpha-synuclein)-transformed Parkinson's disease mouse models and the efficacy was evaluated. The test drug was prepared using 10% NMP, 40% PEG 400, and 50% D.W. as carriers for the compounds in the embodiment.

## Experimental Example 10.2. Experimental design and mouse weight changes

FIG. 8 is a schematic diagram of an experiment of an efficacy experiment on a mouse model of α-Syn transformed Parkinson's disease.

Mice aged 4 months were divided into 7 groups as shown in Table 10 below. The test drug was administered once daily (QD) via oral administration (PO) with a solution of 5 mL/kg for 5 months from 4 months to 9 months of age. Motor behavior tests (beam test) and neurological scores (modified Irwin test) were performed at 4 months (before administration), 7 months, and 9 months. After the final behavioral test, tissue samples were taken. Weight was measured on the same day once a week from 4 months of age until the end of the study. Mice with significantly worse general health were excluded from the study.

[Table 10]

| Group | Number of Animals | Type | Treatment Drug | Route of Administration | Dose |
|---|---|---|---|---|---|
| G1 | 8 | WT | - | - | - |
| G2 | 8 | TG | Carrier | PO, QD | - |
| G3 | 8 | TG | Memantine | PO, QD | 10 mg/kg |
| G4 | 8 | TG | Example 3 | PO, QD | 2 mg/kg |
| G5 | 8 | TG | Example 3 | PO, QD | 10 mg/kg |
| G6 | 8 | TG | Example 3 | PO, QD | 50 mg/kg |
| G7 | 8 | TG | Example 2 | PO, QD | 2 mg/kg |

FIG. 9 is a graph of the weight change by group in a α-Syn transgenic Parkinson's disease mouse model. Referring to FIG. 9, it was confirmed that the weight of the WT mouse (G1) increased over time, while the weight gain of the TG group (G2-7) was stagnant. The difference in body weight between WT mice and TG mice became evident after about 21 to 22 weeks of age, and it was confirmed that drug administration did not have a significant effect on the weight of TG mice.

**Experimental example 10.3. Neurological behavior improvement effect**

At 4 months of age (pre-dose, baseline), 7 months, and 9 months, the neurological disturbance behaviors listed in Table 11 below were observed for 1 to 2 min per mouse. Scores were recorded by direct observation of mice in cages for neurological behavioral disorders, except for startle reflex, tail pinch, and righting reflex. To test the startle reflex, a small remote control was used to make loud noises and check behaviors such as jumping, stopping, and blinking quickly. For the stereotactic reflex, each mouse was removed from the cage and placed with its back facing the floor so that the mouse could correct its posture on its own. The tail pinch reflex was tested by gently squeezing the tip of the tail with forceps.

A score of 0 was given if there were normal or no abnormal features such as motor activity, 1 point if mild abnormalities were observed, and 2 to 3 points were given if serious abnormalities were observed. The scores for all observed behaviors for each mouse were added, and the average of the added scores for each group was calculated.

[Table 11]

| # | Neurological score indicator | # | Neurological score indicator |
|---|---|---|---|
| 1 | Head tremor | 16 | Seizure |
| 2 | Head twitch | 17 | Urine staining |
| 3 | Head bobbing | 18 | Lacrimation |
| 4 | Head searching | 19 | Salivation |
| 5 | Body tremor | 20 | Limb splay |
| 6 | Body twitch | 21 | Catalepsy |
| 7 | Tail tremor | 22 | Abnormal gait |
| 8 | Tail twitch | 23 | Tip toe walking |
| 9 | Tail Straub tail | 24 | Slow, careful movements |
| 10 | Piloerection | 25 | Excessive grooming |
| 11 | Shallow respiration | 26 | Circling, sniffing |
| 12 | Flattened body posture | 27 | Chewing |
| 13 | Swollen face | 28 | Loss of startle reflex (jumping, freezing, eye blinking) |
| 14 | Drooping eyelids (Ptosis) | 29 | Loss of righting reflex |
| 15 | Irritability | 30 | Loss of response to tail pinch |

FIG. 10 is a graph of the group-specific neurological behavior scores of α-Syn transformed Parkinson's disease

mouse models. At 4 and 7 months of age, the neurological score (TG + transporter) of G2 was significantly lower compared to G1. The group administered embodiment 3 or embodiment 2 significantly reduced the neurological score of TG mice compared to G2. The neurological scores of groups G4 to G7 remained very low overall throughout the study period, and it was confirmed that there was no neurological toxicity caused by the drug.

**Experimental Example 10.4. Evaluation of the efficacy of improving step error (beam test)**

The procedure described by Fleming et al. (J Neurosci, 24:9434-40, 2004) was adopted as the beam test (Challenging Beam Traversal). Exercise capacity was measured with a new beam test that was a modification of the existing beam walking test. The beam is made of Plexiglas and consists of four sections (each 25 cm and a total length of 1 m), each with a different width. The beam is designed to start at 3.5 cm wide and gradually narrow in increments of 0.5 cm × 1 cm. A shelf 1 cm wide was placed 1.0 cm below the top surface of the beam.

The mouse was trained to cross the length of the beam, starting from the widest section and ending with the narrowest and most difficult section. The narrow end of the beam is designed to connect directly to the mouse's house cage. The mice were trained for two days before testing, and all training was carried out without a mesh grid. On the first day, two auxiliary exercises were performed, in which the mouse was placed on a beam and the home cage was placed close to the mouse. This induces it to move forward along the beam. After two auxiliary trainings, the mouse was able to traverse the entire length of the beam unaided. On the first day of training, it was confirmed that all mice completed five unassisted runs over the entire length of the beam. On the second day of training, mice performed five tests. On the day of the test, a mesh grid (1 cm square) of the corresponding width was placed on the beam surface to increase the difficulty, and a space of about 1 cm was placed between the grid and the beam surface. The lower shelf provided a support for the mouse to use when the limb slipped off the grid, allowing the mouse to evaluate without the need to use a compensatory motor strategy to complete the test. A total of 5 times were filmed on video of the mouse crossing the grid beam.

Videotape analysis was performed by researchers who did not know the mouse genotype/group to check the slow motion for error, the number of steps taken by each mouse, and the transverse time over the five experiments were evaluated. The error was counted if the limb (front or hind leg) passed through the grid during forward movement and was visible between the grid and the beam surface. Individual mice could slide up to four times per step. Error was measured by scoring each limb slippage individually. For example, a mouse with three limbs slipping off the grid in one step was rated with an error score of 3 points, while a mouse with only one limb slipped off the grid in one step was evaluated with an error score of 1 in that step. If the mouse did not move forward, or if the mouse's head was facing the left or right of the beam, it did not count as slippage. The number of step errors and the time it took to cross were measured and averaged in all five experiments.

FIG. 11 is a graph showing the results of the assessment of gait errors by group in a α-Syn transgenic Parkinson's disease mouse model. Referring to FIG. 11, group G2 made significantly more errors during traversal compared to group G1 in beam testing. The memantine (G3), Example 3 (G4 to G6) or Example 2 (G7) treatment group had a significant reduction in the number of step errors compared to Group G2 at 7 and 9 months. Through this, it was confirmed that the embodied compound improves the behavior error.

**Experimental Example 10.5. Evaluation of efficacy in improving dopaminergic neurodegeneration**

At 9 months of age, the measurement period ended, the mice were anesthetized, and then the experiment was terminated by cardiac puncture according to IACUC guidelines. Mice were perfused transcardially with 0.9% saline for tissue staining, followed by 4% paraformaldehyde until the mice were fixed. The brains were isolated and immobilized by soaking in 4% paraformaldehyde at 4°C for 24 hours, followed by 30% sucrose added to PBS containing 0.02% sodium azide and stored at 4°C for 3 days. Brain samples embedded in OCT compounds were sectioned to a thickness of 20 μm using Leica CM1950 (Leica Microsystems, Germany). The sections were stored at 4 °C in a preservation solution until they were stained by antibodies.

The brain tissue was washed with PBS for 10 minutes three times while gently shaking the tissue. The tissue was then reacted with PBS, 10% normal chlorine serum, and a barrier buffer containing 0.3% Triton X-100 for 1 h at room temperature. The tissues were incubated overnight at 4 °C with the primary antibody (Iba-1, TH) according to the antibodies and dilution rates in the table below. After primary antibody culture, the tissue was washed with PBS solution and reacted with the secondary antibody for 1 h at room temperature. Subsequently, the sections were washed three times for 10 min each with PBS solution and mounted using DAPI mounting solution (VECTASHIELD, #H1500) on the slides and coverslips.

[Table 12]

| Antibody | Supplier | # CAT | Dilution Rate |
|---|---|---|---|
| anti-tyrosine hydroxylase | Abcam | Ab112 | 1/1000 |
| anti-Iba1 | Abcam | Ab283319 | 1/250 |

TH-stained brain tissue was imaged using an immunofluorescence microscope (Olympus, BZ21) in Olympus. The fluorescence intensity of the striatum was analyzed using Image J software.

FIG.s 12a and 12b are graph and immunofluorescence analysis images showing the results of the evaluation of the efficacy of improving dopaminergic neurodegeneration by group in a mouse model of α-Syn transformed Parkinson's disease. Referring to FIG. 12a and FIG. 12b, the TH staining intensity of group G2 striatum tended to be lower than that of group G1. The TH staining intensity of the non-memantine embodiment 3 or embodiment 2 (groups G4 to G7) was significantly higher than that of group G2. The TH staining intensity of group G7 (TG + Example 2) was also significantly higher than that of group G2. As a result, it was confirmed that the embodied compound can significantly improve dopaminergic neurodegeneration.

**Experimental Example 10.6. Evaluation of microglial reduction efficacy**

To assess microgliosis of the cortex, Iba-1-stained brain tissue was imaged in experimental example 10.5 using an immunofluorescence microscope (Olympus, BZ21) in Olympus. Each marker was manually counted to quantify Iba1.

FIG. 13a and FIG. 13b are graph and immunofluorescence assay images showing the results of the evaluation of microglial reduction efficacy by group in a mouse model of α-Syn transformed Parkinson's disease. Referring to FIG. 13a and FIG. 13b, the number of IBA-1 positive cells in the cerebral cortex was significantly reduced in the memantine (G3), Example 2 mg/kg and 10 mg/kg (G4 and G5) and Example 2 mg/kg (G7) administration groups. Through this, it was confirmed that the Example compounds significantly reduced microglia.

**Experimental Example 11. Evaluation of the Efficacy of SOD1 G93A on a Mouse Model of Transgenic Amyotrophic Lateral Sclerosis**

**Experimental Example 11.1. Preparing for the experiment**

Experimental F1 mice were generated by crossing G93A SOD1 (high-copy SOD1-G93A, 25 TG copy G1H, stock number 002726) males with B6SJL strain background (non-carrier, WT) females at Jackson Labs. All mice were raised in a normal light and dark cycle (08:00-20:00) in an environment with controlled temperature (22±1°C) and humidity (30-50%), and were provided with unlimited food and water (Ad libitum). At the age of 15~21 days, ear markings were made, ear or tail samples were taken, and PCR analysis for genotyping was performed. In addition, tail samples were collected at the end point for genotyping if necessary.

The test drug was administered to normal mice and a mouse model of SOD1 G93A modified amyotrophic lateral sclerosis (ALS) and its efficacy was evaluated. The test drug was prepared using 10% NMP, 40% PEG 400, and 50% D.W. as carriers for the compounds in the embodiment.

**Experimental Example 11.2. Design of experiments**

FIG. 14 is a schematic diagram of an experiment for an efficacy experiment on a SOD1 G93A transgenic ALS mouse model.

Mice at 8 weeks of age were divided into three groups as shown in Table 13 below. The test drug was administered once daily (QD) via oral administration (PO) in a solution of 5 mL/kg from 8 weeks of age until death. Body weight was measured weekly, and disease score was measured daily from 8 weeks until the death of the animal. Motor behavior tests (grip strength) and neurological score tests (modified Irwin test) were performed at 9, 11, 13, 15, 17, 19, and 21 weeks, respectively. The survival of the mice was monitored daily. If the overall health of the mice deteriorated significantly, they were excluded from the study.

[Table 13]

| Group | Number of Animals | Type | Treatment Drug | Route of Administration | Dose |
|---|---|---|---|---|---|
| G1 | 12 | WT | - | - | - |
| G2 | 12 | TG | Carrier | PO, QD | - |

(continued)

| Group | Number of Animals | Type | Treatment Drug | Route of Administration | Dose |
|---|---|---|---|---|---|
| G3 | 12 | TG | Example 3 | PO, QD | 30 mg/kg |

FIG. 15 is a graph of the change in body weight by group in a SOD1 G93A transgenic ALS mouse model. Referring to FIG. 15, the weight of the Tg group (G2) was significantly lower than that of the WT group (G1). On the other hand, there was no significant difference between the TG (G2) and the drug treatment group (G3), and it was confirmed that the embodied compound did not significantly reduce body weight.

**Experimental Example 11.3. Evaluating the Efficacy of Improving Neurological Behavior**

Neurological disorder behavior was observed for 1 to 2 min per mouse at 9, 11, 13, 15, 17, 19, and 21 weeks of age in the same manner as in Example 10.3.

FIG. 16 is a graph of the group-specific neurological scores of SOD1 G93A transgenic ALS animal models. Referring to FIG. 16, the neurological score of the WT group (G1) was found to be very low, less than 1 throughout the study period. After 13 weeks, the neurological score of the TG group was significantly higher than that of the G1 group. From the 19th week of age onwards, the group (G3) treated with embodiment 3 showed a significant decrease in neurological scores compared to G2. Through this, it was confirmed that the embodied compound significantly improved neurological behavior.

**Experimental Example 11.4. Evaluating the efficacy of improving disease symptoms**

From 8 weeks of age, the neurological scoring system developed by the ALS Therapy Development Institute (ALSTDI) method was used to evaluate disease symptoms in mice. Both hind legs were scored based on Table 14 below.

[Table 14]

| Score | Criteria |
|---|---|
| 0 | When the mouse is hung by the tail, the hind legs are fully extended away from the lateral midline |
| 1 | Hind legs tremble when hanging with the tail |
| 2 | When hanging with the tail, both hind feet are caught facing each other |
| 3 | Stumbling and falling when walking |
| 4 | Shut down one or both of your hind legs when walking |
| 5 | Inability to walk (inability to correct itself within 30 seconds when placing the mouse with its back facing the floor) |

FIG. 17 is a graph of the change in disease scores by group in a mouse model of SOD1 G93A transgenic ALS. Referring to FIG. 17, the disease incidence score in the TG group (G2 and G3) increased significantly compared to the WT group (G1), and the difference was marked at approximately 15 weeks of age. The group treated with Example 3 (G3) tended to have a lower disease symptom score compared to G2 during the observation period, and decreased significantly at 20 weeks. Through this, it was confirmed that the embodied compound significantly improved disease symptoms.

**Experimental Example 11.5. Evaluation of grip strength improvement efficacy**

Grip strength measurements were performed on mice aged 9, 11, 13, 15, 17, 19, and 21 weeks of age. The mouse was placed on a grip strength measurement device (San Diego Instruments, San Diego, USA) in such a way that the mouse grabbed the handle of a small mesh with its front paws. I put the mouse down on the platform and then slowly pulled the handle with my tail until the mouse released the handle. The device automatically measured the grip strength of the mouse in grams. Five scores per mouse were recorded consecutively in a one-day session.

FIG. 18 is a graph of the group-specific changes in grip strength in a SOD1 G93A transgenic ALS mouse model. Referring to FIG. 18, grip strength in the TG group generally decreased over time and was significantly lower than in group G1. The group (G3) that treated embodiment 3 tended to increase grip strength compared to group G2, and the grip strength increased significantly from 19 weeks. Thus, it was confirmed that the embodied compound significantly improved grip strength.

### Experimental Example 11.1. Evaluation of survival improvement efficacy

The survival of the mice was monitored daily. The survival age of mice was recorded and labeled as Kaplan Meier survival plots.

FIG. 19 is a graph of the survival rate by group of SOD1 G93A transgenic ALS mouse models. Referring to FIG. 19, the survival rate of the TG group began to decrease between about 110 and 120 days of age, and the group (G3) treated with embodiment 3 showed a tendency to improve survival rate, although it was not significant compared to group G2. As a result, it was confirmed that the embodied compound improved the survival rate.

### Experimental Example 12. Evaluation of Efficacy on Reserpine-Induced Fibromyalgia Rat Model

### Experimental Example 12.1. Preparing for the experiment

A 6-week-old Sprague Dawley (SD) male rat was used. All rats were raised in a normal light and dark cycle (08:00-20:00) in an environment with controlled temperature (22±1°C) and humidity (30-50%), and were provided with unlimited food and water (Ad libitum).

The test drug was administered to normal rats and Reserpine-induced fibromyalgia (Fibromyalgia) rat models and the efficacy was evaluated. The test drug was prepared using 10% NMP, 40% PEG 400, and 50% D.W. as carriers for the compounds in the embodiment.

### Experimental example 12.2. Design of experiments

FIG. 20 is a schematic diagram of an experiment for an efficacy trial on a Reserpine-induced fibromyalgia rat model.

Reserpine-induced fibromyalgia model generation and drug administration were carried out on 6-week-old male SD rats divided into 6 groups as shown in Table 15 below: control group (G1), reserpine + delivery (G2), reserpine + gabapentin 50 mg/kg (G3), reserpine + Example 2 3 mg/kg (G4), 10 mg/kg (G5), and 30 mg/kg (G6). To create a rat fibromyalgia model, reserpine (Sigma-aldrich, 1 mg/kg) dissolved in 0.5% acetic acid was administered subcutaneously once for 3 days from 3 days before the start of the experiment. Beginning on Day 1, the once-daily (QD) delivery body, gabapentin, and embodiment2 were administered intraperitoneally (IP) or orally (PO) 2 hours before the experiment was performed.

[Table 15]

| Group | Number of Animals | Type | Treatment Drug | Route of Administration | Dose |
|---|---|---|---|---|---|
| G1 | 6 | Saline solution | - | - | - |
| G2 | 6 | Reserpine | Carrier | PO, QD | - |
| G3 | 6 | Reserpine | Gabapentin | IP, QD | 50 mg/kg |
| G4 | 6 | Reserpine | Example 2 | PO, QD | 3 mg/kg |
| G5 | 6 | Reserpine | Example 2 | PO, QD | 10 mg/kg |
| G6 | 6 | Reserpine | Example 2 | PO, QD | 30 mg/kg |

### Experimental Example 12.3. Evaluation of pain improvement efficacy

The right hind foot tactile response test (von Frey test) was performed 5 times (before the fibromyalgia model, and on the 1st, 2nd, 4th and 6th days after the model was generated). Before all behavioral tests, rats were accustomed to the environment for 30 minutes, and all experimenters performing behavioral tests were made blind to drug administration information. Before model generation, baseline measurements were performed by performing the von Frey test on all rats. The rat was placed on a wired mesh and placed in a transparent plastic box (20 x 20 x 14 cm), and the foot withdrawal threshold was measured by applying a von Frey filament (ascending weight: 0.4, 0.6, 1.4, 2, 4, 6, 8, and 15 g, North Coast) to the middle of the right hind foot to assess foot allodynia.

FIG. 21 is a graph of the results of the evaluation of the pain-improving efficacy for the Reserpine-induced fibromyalgia rat model. Compared with the control group (G1), the reserpine treatment group (G2) showed high sensitivity, and after model generation, it was confirmed that the sensitivity of the gabapentin treatment group (G3) and the embodiment 2 treatment group (G4 to G6) gradually improved over time. Example 2 showed a tendency to recover

dose-dependently, showing significant efficacy at 30 mg/kg. Through this, it was confirmed that the compound in the embodiment significantly improved pain.

### Experimental Example 13. Evaluation of Efficacy in a Mouse Model of CDAHFD-Induced Nonalcoholic Steatohepatitis

#### Experimental example 13.1. Preparing for the experiment

A 3-week-old C57BL/6 male mouse (Koatech, Korea) was purchased. Mice were raised in groups of up to 5 per cage in a normal light and dark cycle (08:00~20:00) in a controlled environment with temperature (22±1°C) and humidity (30-50%), and were provided with unlimited food and water (Ad libitum). The mice were allowed to adapt for one week before the start of the experiment.

The test drug was administered to normal mice and CDAHFD (A choline-deficient, L-amino acid-defined, high-fat diet)-induced non-alcoholic steatohepatitis (NASH) mouse models and evaluated its efficacy. The test drug was prepared using 10% NMP, 40% PEG 400, and 50% D.W. as carriers for the compounds in the embodiment.

CDAHFD was formulated as a high-fat diet containing 0.1% methionine and 60 kcal% fat, and deficient in choline. C57BL/6J mouse models fed CDAHFD develop rapidly steatohepatitis with fibrosis in a short period of time.

#### Experimental example 13.2. Design of experiments

FIG. 22 is a schematic diagram of an experiment of an efficacy experiment on a mouse model of CDAHFD-induced non-alcoholic steatohepatitis.

Creation of CDAHFD-induced NASH model and drug administration were carried out by dividing C57BL/6 male mice at 3 weeks of age into 7 groups as shown in Table 16 below. The control group (G1) was fed standard feed and water. Groups G2 to G7 were fed the CDAHFD (Research Diet Inc, #A06071302) diet for 4 and 8 weeks. Except for the control group, each group received the (QD) delivery orally, Selonsertib 30 mg/kg, embodiment 3 10 mg/kg, embodiment 3 30 mg/kg, embodiment 2 10 mg/kg, and embodiment 2 30 mg/kg orally from 5 weeks of age.

[Table 16]

| Group | Number of Animals | Type | Treatment Drug | Route of Administration | Dose |
|---|---|---|---|---|---|
| G1 | 10 | Standard feed | - | - | - |
| G2 | 10 | CDAHFD | Carrier | PO, QD | - |
| G3 | 10 | CDAHFD | Selonsertib | PO, QD | 30 mg/kg |
| G4 | 10 | CDAHFD | Example 3 | PO, QD | 10 mg/kg |
| G5 | 10 | CDAHFD | Example 3 | PO, QD | 30 mg/kg |
| G6 | 10 | CDAHFD | Example 2 | PO, QD | 10 mg/kg |
| G7 | 10 | CDAHFD | Example 2 | PO, QD | 30 mg/kg |

#### Experimental Example 13.3. Evaluation of efficacy in improving liver fibrosis

Picrosirius red staining was used for histological visualization of collagen fibers. Histopathology analysis evaluated the efficacy of improving liver fibrosis.

For mice in each group, three sections of the liver fixed with 4% PFA (paraformaldehyde) were prepared with paraffin blocks. Paraffin blocks were sectioned to a thickness of 5 μm (Histocore AUTOCUT R, Leica Microsystems, Germany) and the sections were mounted on silane-coated slides (MUTO, JPN) and stored until staining.

Xylene was used to remove paraffin from the slides, and the slides were moisturized and washed. The slides were stained with a solution of Picrosirius red (ab150681, Abcam, UK) and washed off with a solution of acetic acid. The slides were fitted after dehydration and cleaning using alcohol and xylene. For each section, two images of different areas were taken using a microscope (Axioscan7, ZEISS, Germany). The fibrotic area was measured using image J software compared to the total area.

FIG.s 23a and 23b are graphs and images of Picrosirius red stained area ratios in the liver of a CDAHFD-induced mouse model of nonalcoholic steatohepatitis. Referring to FIG.s 23a and 23b, the CDAHFD group had a significantly higher relative area stained with Picrosirius red compared to group G1, indicating an appropriately formed NASH model. The Picrosirius red staining area was significantly reduced in the comparator drug Selonsertib (G3), embodiment 3 (G4

and G5), and embodiment 2 (G6 and G7), and the most improved efficacy was confirmed in the 30 mg/kg group of Example 2 after 4 weeks of the CDAHFD diet. This confirmed that the embodied compound significantly improved liver fibrosis.

## Claims

1. A compound represented by Chemical Formula 1, a stereoisomer, a solvate, a hydrate, or a pharmaceutically acceptable salt thereof:

[Chemical Formula 1]

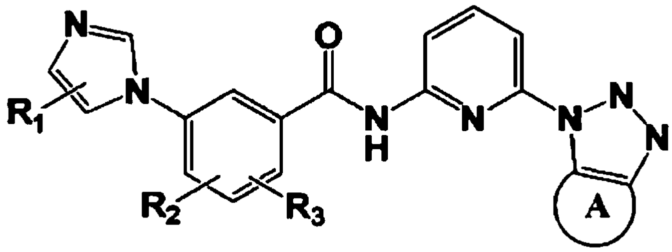

wherein,

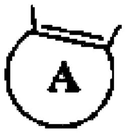

is a substituted or unsubstituted phenyl or substituted or unsubstituted $C_{3-8}$ cycloalkenyl;

wherein, the substituted phenyl and the substituted $C_{3-8}$ cycloalkenyl are each independently substituted with $C_{1-6}$ alkyl, $C_{1-6}$ alkoxy or halogen; and
$R_1$, $R_2$ and $R_3$ are each independently H, $C_{1-6}$ alkyl, halogen, $C_{3-6}$ cycloalkyl or halo $C_{1-6}$ alkyl.

2. The compound represented by Chemical Formula 1, a stereoisomer, a solvate, a hydrate, or a pharmaceutically acceptable salt thereof according to claim 1, wherein

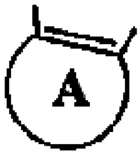

is a substituted or unsubstituted phenyl or substituted or unsubstituted $C_{5-8}$ cycloalkenyl,

wherein, the substituted phenyl and the substituted $C_{3-8}$ cycloalkenyl are each independently substituted with $C_{1-3}$ alkyl, $C_{1-3}$ alkoxy or halogen; and
$R_1$, $R_2$ and $R_3$ are each independently H, $C_{1-3}$ alkyl, halogen, $C_{3-6}$ cycloalkyl or halo $C_{1-3}$ alkyl.

3. The compound represented by Chemical Formula 1, a stereoisomer, a solvate, a hydrate, or a pharmaceutically acceptable salt thereof according to claim 1, wherein

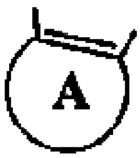

is $C_{5-8}$ cycloalkenyl, which contains a phenyl or a double bond;
wherein,

$R_1$ is $C_{3-6}$ cycloalkyl;
$R_2$ is $C_{1-3}$ alkyl; and
$R_3$ is halogen.

**4.** The compound represented by Chemical Formula 1, a stereoisomer, a solvate, a hydrate, or a pharmaceutically acceptable salt thereof according to claim 1, wherein

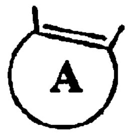

is $C_{5-8}$ cycloalkenyl containing a phenyl or a double bond;

wherein, $R_1$ is cyclopropyl;
$R_2$ is methyl; and
$R_3$ is fluorine.

**5.** The compound represented by Chemical Formula 1, a stereoisomer, a solvate, a hydrate, or a pharmaceutically acceptable salt thereof according to claim 1, wherein the compound represented by Chemical Formula 1 is selected from the group consisting of:

<1> N-(6-(1H-benzo[d][1,2,3]triazole-1-yl)pyridine-2-day)-5-(4-cyclopropyl-1H-imidazole-1-day)-2-fluoro-4-methylbenzamide;
<2> 5-(4-cyclopropyl-1H-imidazole-1-day)-N-(6-(5,6-dihydrocyclopenta[ d][1,2,3]triazole-1(4 h)-day)pyridine-2-day)-2-fluoro-4-methylbenzamide;
<3> 5-(4-cyclopropyl-1H-imidazole-1-yl)-2-fluoro-4-methyl-N-(6-(4,5,6,7-tetrahydro-1H-benzo[d][1,2,3]triazole-1-yl)pyridine-2-yl)methylbenzamide;
<4> 5-(4-cyclopropyl-1H-imidazole-1-yl)-2-fluoro-4-methyl-N-(6-(5,6,7,8-tetrahydrocyclohepta[d][1,2,3]triazole-1(4H)-yl)pyridine-2-yl)benzamide; and
<5> 5-(4-cyclopropyl-1H-imidazole-1-yl)-2-fluoro-N-(6-(4,5,6,7,8,9-hexahydro-1 H-cycloocta[d][1,2,3]triazole-1-yl)pyridine-2-yl)-4-methylbenzamide.

**6.** The compound represented by Chemical Formula 1, a stereoisomer, a solvate, a hydrate, or a pharmaceutically acceptable salt thereof according to claim 1, wherein the compound inhibits ASK1.

**7.** A method for preparing a compound represented by Chemical Formula 1 according to claim 1 comprising:

reacting a compound represented by Chemical Formula 2 and a compound represented by Chemical Formula 3 to prepare a compound represented by Chemical Formula 1 as shown in Reaction Equation 1,

[Reaction Equation 1]

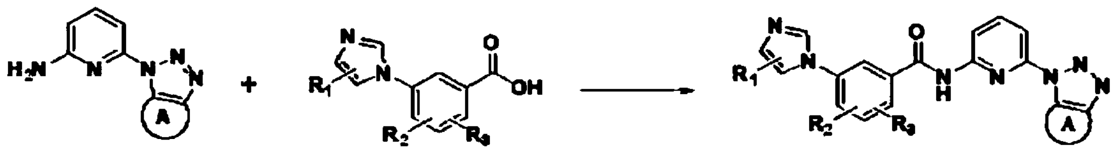

2 3 1

wherein,

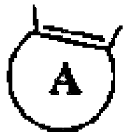,

$R_1$, $R_2$, and $R_3$ are same as defined in Chemical Formula 1 according to claim 1.

**8.** A pharmaceutical composition comprising as active an active ingredient a compound represented by Chemical Formula 1, a stereoisomer, a solvate, a hydrate, or a pharmaceutically acceptable salt thereof according to claim 1.

**9.** A pharmaceutical composition for preventing or treating an ASK1-related disease comprising the compound represented by chemical formula 1, a stereoisomer, a solvate, a hydrate, or a pharmaceutically acceptable salt thereof according to claim 1 as an active ingredient.

**10.** The pharmaceutical composition according to claim 9, wherein the ASK1-related disease is neurodegenerative diseases, cardiovascular diseases, autoimmune diseases, or liver diseases.

**11.** The pharmaceutical composition according to claim 10, wherein the neurodegenerative disease is one of more selected from the group consisting of Alzheimer's disease, hippocampal sclerosis, frontotemporal dementia (FTD), frontotemporal degeneration (FTLD), Huntington's disease, corticobasal degeneration, amyotrophic lateral sclerosis, spinal muscular atrophy, motor neuron disease, inclusion body myositis, Parkinson's disease, Lewy body dementia, Lewy body disease, multiple system atrophy, progressive supranuclear palsy, Peak disease, Prion disease, traumatic brain injury, ischemic and hemorrhagic stroke, cerebral ischemia, hypoxia, or glutamate neurotoxicity.

**12.** The pharmaceutical composition according to claim 10, wherein the cardiovascular disease is one or more selected from the group consisting of heart failure, ischemia, recurrent ischemia, myocardial infarction, arrhythmia, acute coronary syndrome, diabetes, atherosclerosis, and intermittent claudication.

**13.** The pharmaceutical composition according to claim 10, wherein the autoimmune disease is one or more selected from the group consisting of rheumatoid arthritis, fibromyalgia, systemic lupus erythematosus, multiple sclerosis, diabetes, systemic sclerosis , Graves' disease, Guillain-Barré syndrome, myasthenia gravis, psoriasis, Crohn's disease, ulcerative colitis, optic neuritis, and Sjögren's syndrome.

**14.** The pharmaceutical composition according to claim 10, wherein the liver disease is one or more selected from the group consisting of non-alcoholic fatty liver (NASH), alcoholic fatty liver, liver fibrosis, liver cancer, hepatotoxicity, cholestasis, cirrhosis, liver ischemia, liver abscess, hepatic coma, and liver atrophy.

**15.** A method for preventing or treating an ASK1-related disease, comprising:
administrating the compound according to any one of claims 1 to 6, or a solvate, a stereoisomer, or a pharmaceutically acceptable salt thereof to a subject.

**16.** A use of the compound according to any one of claims 1 to 6, or a solvate, a stereoisomer, or a pharmaceutically acceptable salt thereof for prevention or treatment of an ASK-1 related disease.

**17.** A use of the compound according to any one of claims 1 to 6, or a solvate, a stereoisomer, or a pharmaceutically acceptable salt thereof for the manufacture of drugs to prevent or treat an ASK1-related disease.

[FIG. 1]

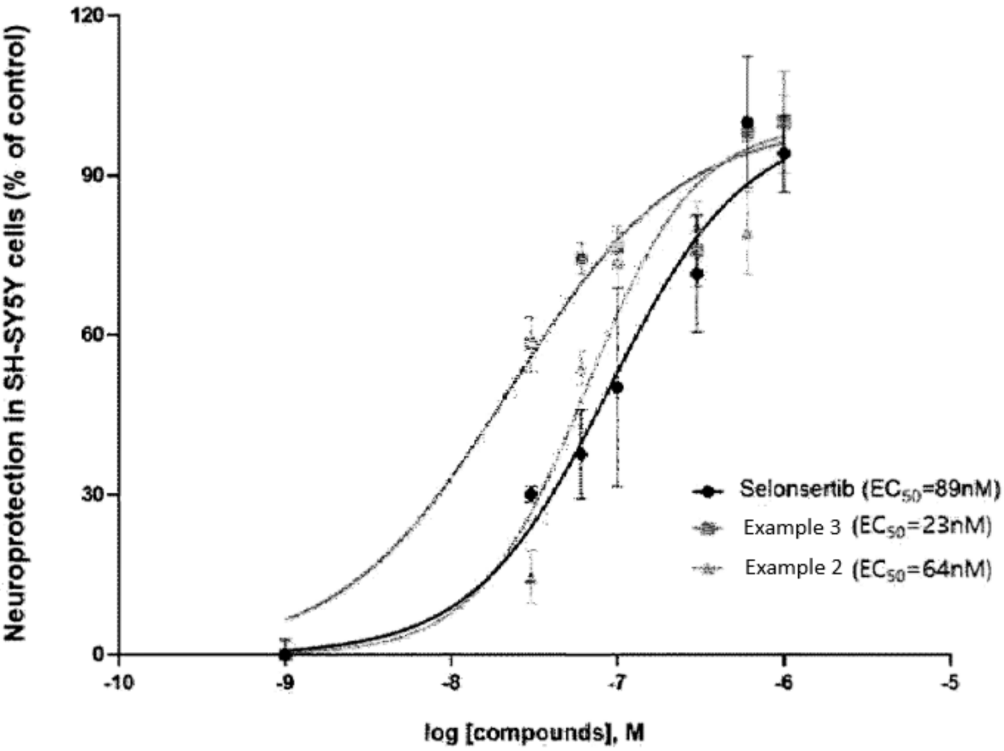
120
90
60
30
0
-10
-9
-8
-7
-6
-5
Neuroprotection in SH-SY5Y cells (% of control)
log [compounds], M
Selonsertib ($EC_{50}$=89nM)
Example 3 ($EC_{50}$=23nM)
Example 2 ($EC_{50}$=64nM)

[FIG. 2]

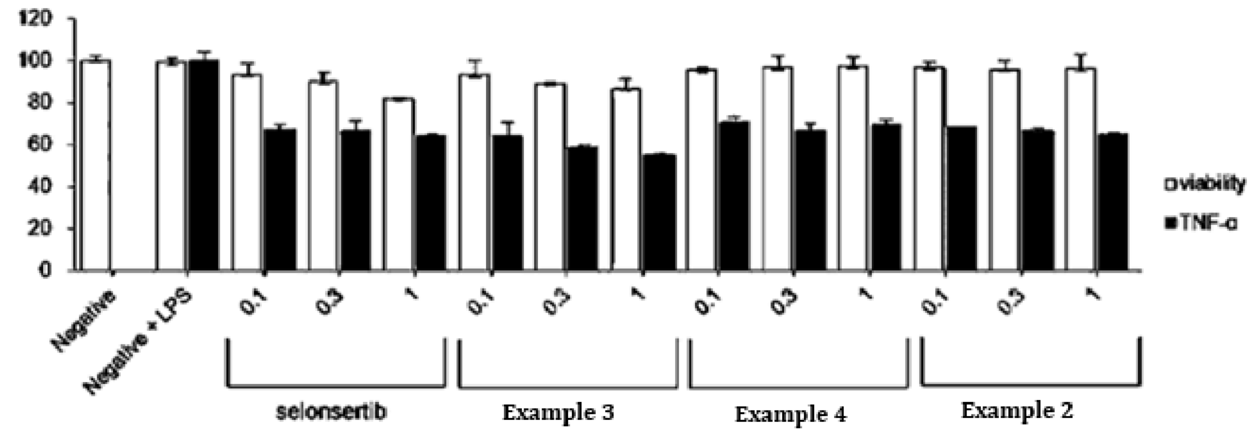
120
100
80
60
40
20
0
Negative
Negative + LPS
0.1
0.3
1
selonsertib
Example 3
Example 4
Example 2
viability
TNF-α

[FIG. 3]

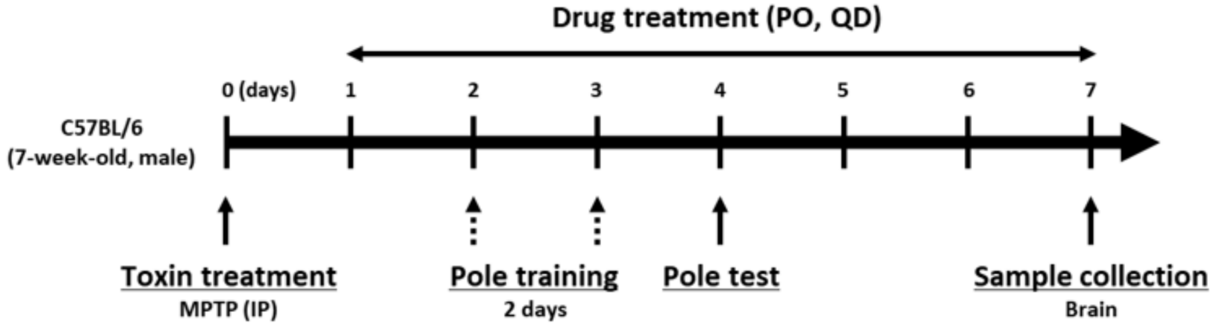

Drug treatment (PO, QD)
0 (days)
1
2
3
4
5
6
7
C57BL/6
(7-week-old, male)
Toxin treatment
MPTP (IP)
Pole training
2 days
Pole test
Sample collection
Brain


[FIG. 4]

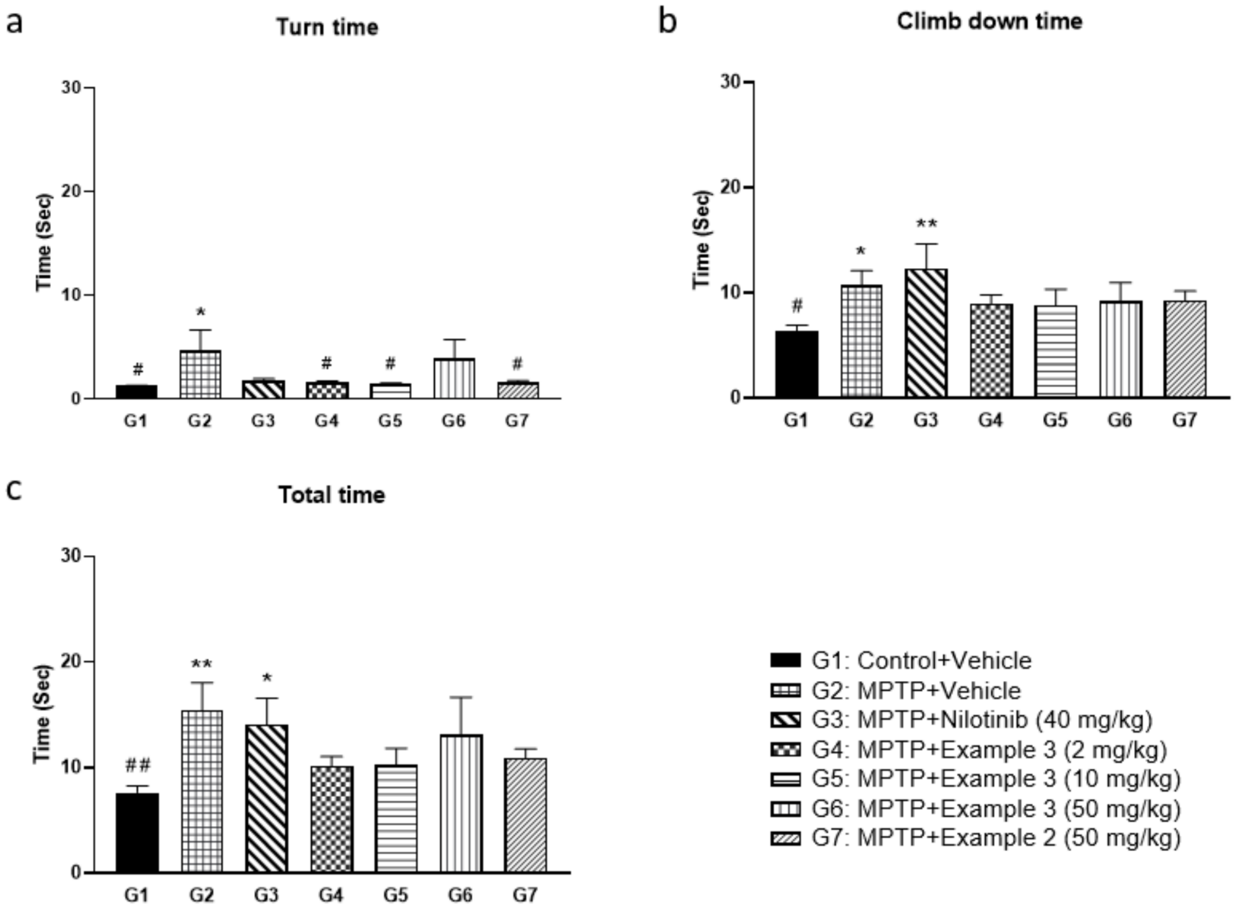

a
Turn time
b
Climb down time
c
Total time
Time (Sec)
G1
G2
G3
G4
G5
G6
G7
G1: Control+Vehicle
G2: MPTP+Vehicle
G3: MPTP+Nilotinib (40 mg/kg)
G4: MPTP+Example 3 (2 mg/kg)
G5: MPTP+Example 3 (10 mg/kg)
G6: MPTP+Example 3 (50 mg/kg)
G7: MPTP+Example 2 (50 mg/kg)

[FIG. 5a]

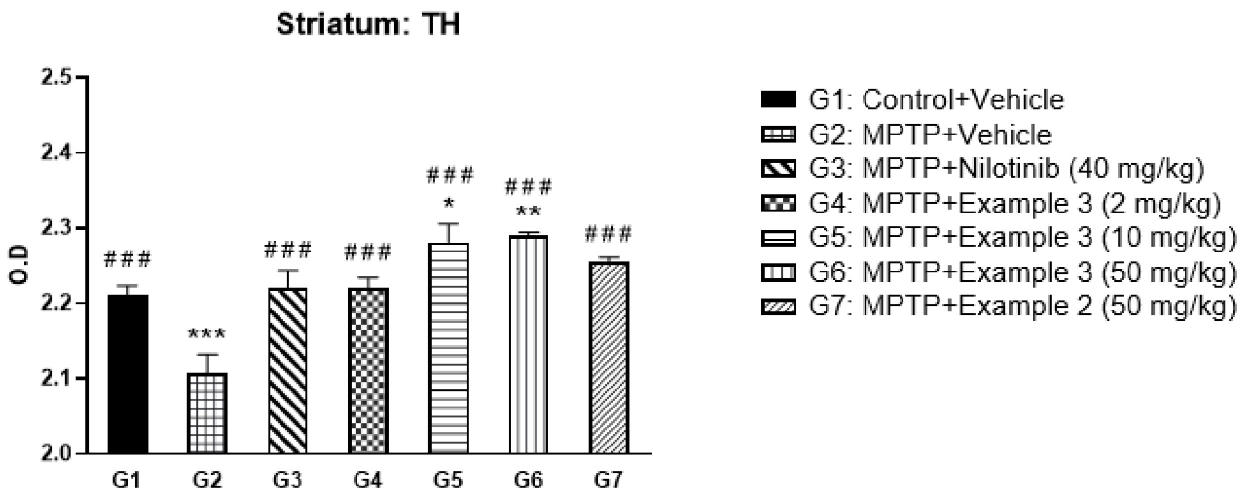
Striatum: TH
O.D
2.5
2.4
2.3
2.2
2.1
2.0
###
***
###
###
###
*
###
**
###
G1
G2
G3
G4
G5
G6
G7
G1: Control+Vehicle
G2: MPTP+Vehicle
G3: MPTP+Nilotinib (40 mg/kg)
G4: MPTP+Example 3 (2 mg/kg)
G5: MPTP+Example 3 (10 mg/kg)
G6: MPTP+Example 3 (50 mg/kg)
G7: MPTP+Example 2 (50 mg/kg)

[FIG. 5b]

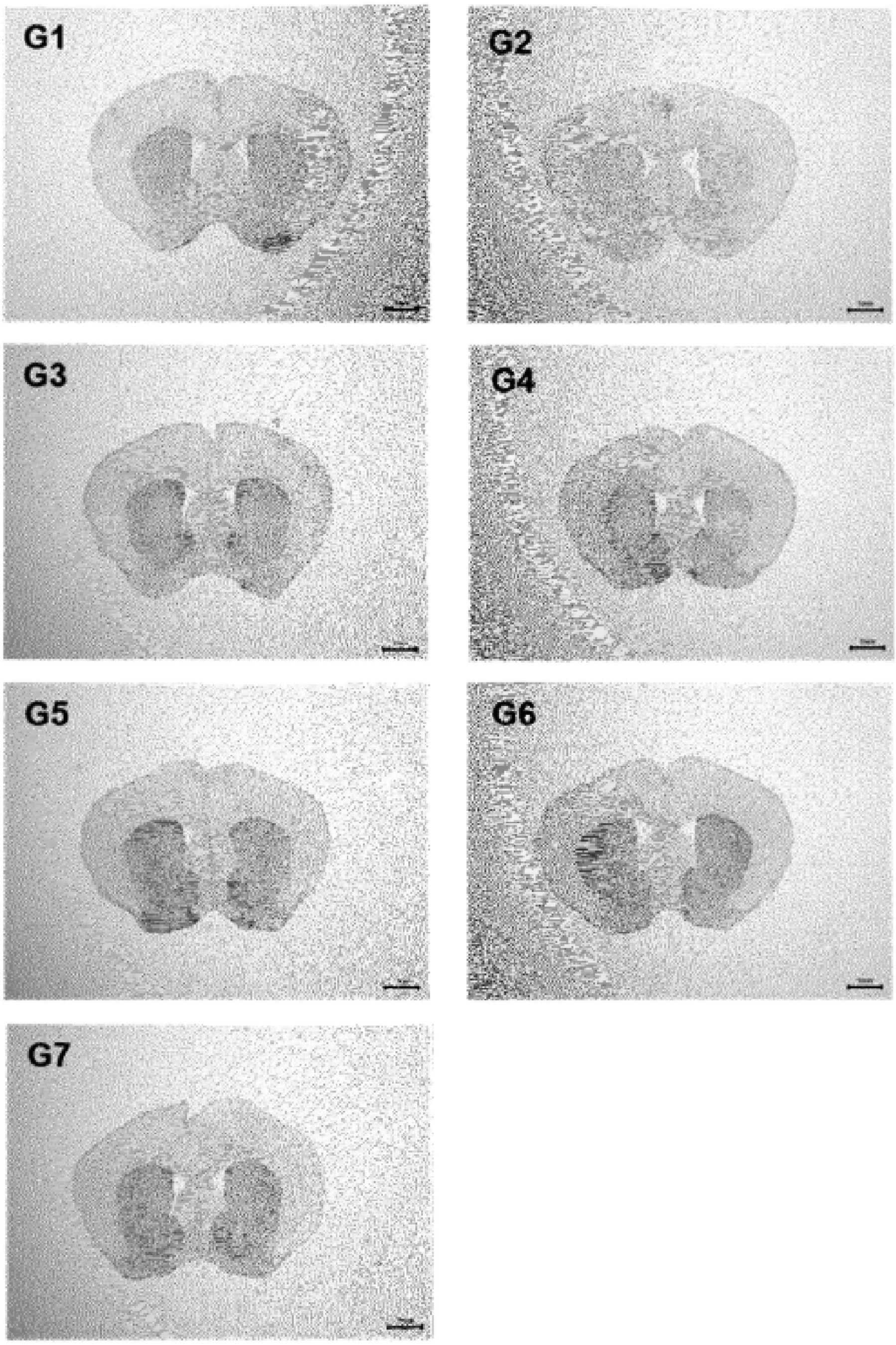

[FIG. 6a]

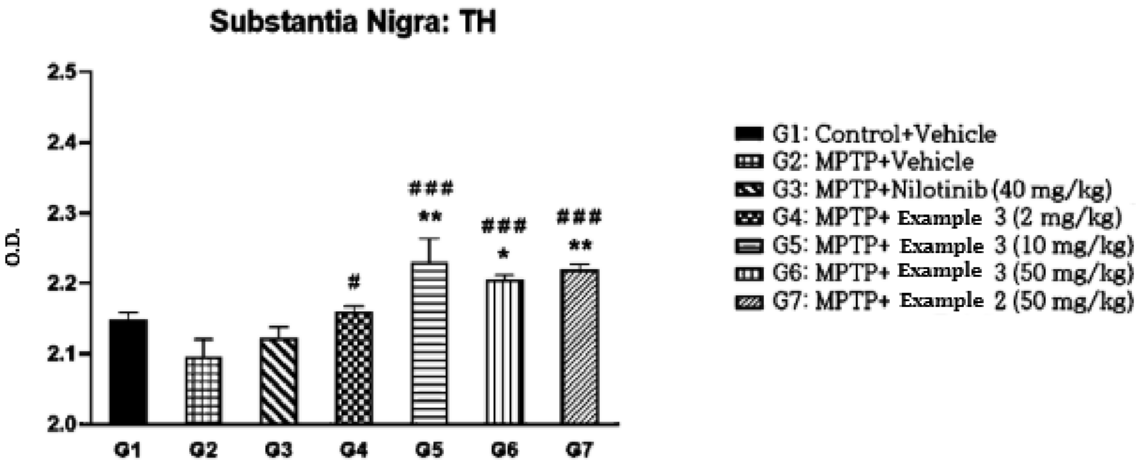
Substantia Nigra: TH
O.D.
2.5
2.4
2.3
2.2
2.1
2.0
G1
G2
G3
G4
G5
G6
G7
#
###
**
###
*
###
**
G1: Control+Vehicle
G2: MPTP+Vehicle
G3: MPTP+Nilotinib (40 mg/kg)
G4: MPTP+ Example 3 (2 mg/kg)
G5: MPTP+ Example 3 (10 mg/kg)
G6: MPTP+ Example 3 (50 mg/kg)
G7: MPTP+ Example 2 (50 mg/kg)

[FIG. 6b]

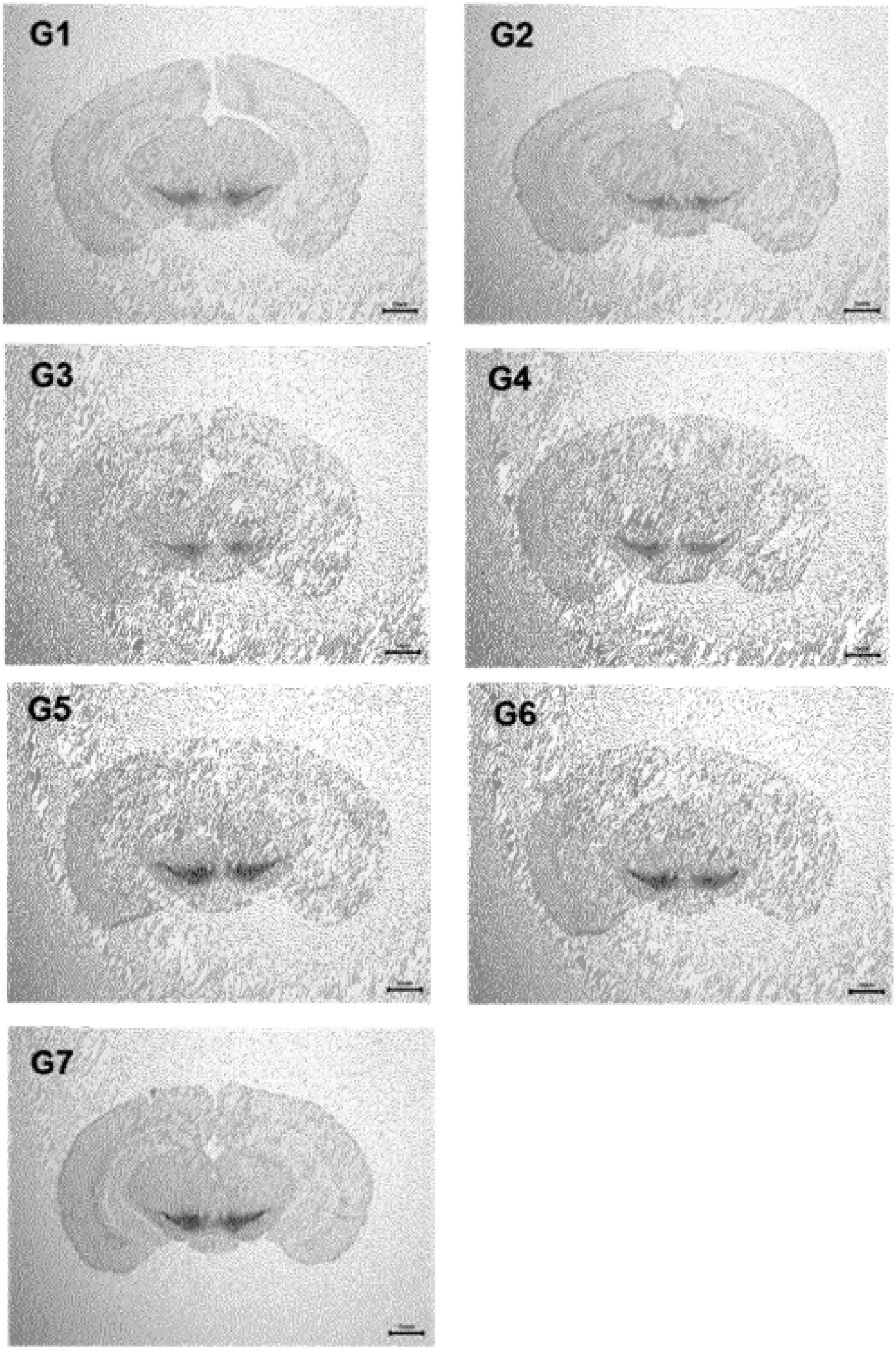

[FIG. 7]

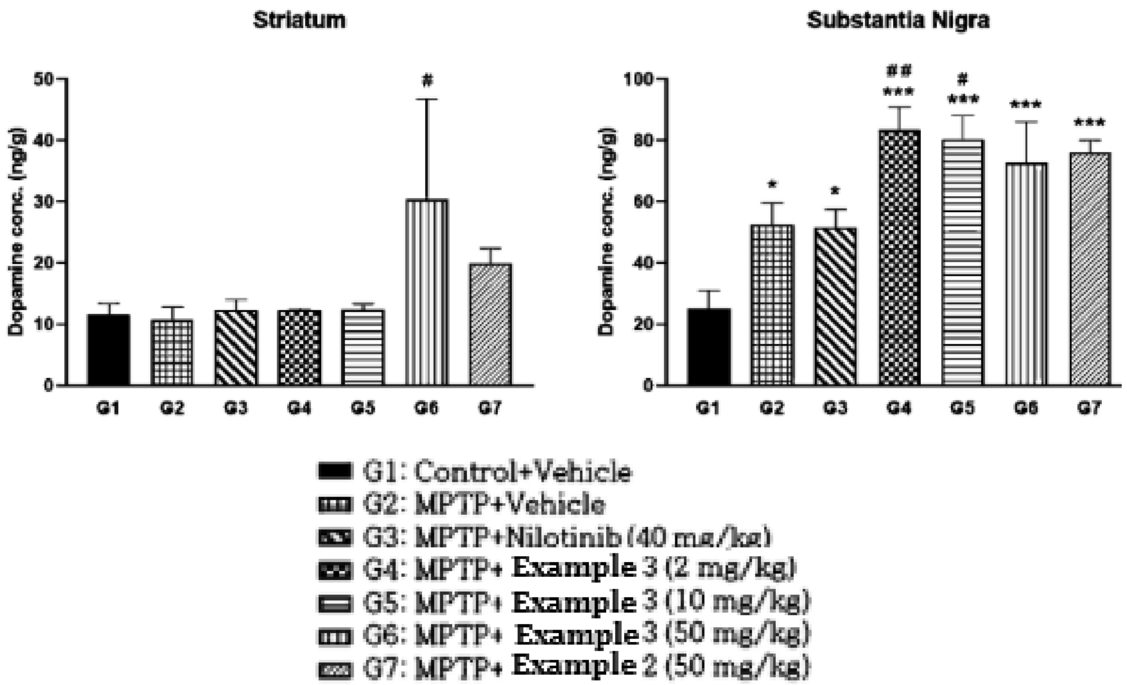
Striatum
Substantia Nigra
Dopamine conc. (ng/g)
G1
G2
G3
G4
G5
G6
G7
G1: Control+Vehicle
G2: MPTP+Vehicle
G3: MPTP+Nilotinib (40 mg/kg)
G4: MPTP+ Example 3 (2 mg/kg)
G5: MPTP+ Example 3 (10 mg/kg)
G6: MPTP+ Example 3 (50 mg/kg)
G7: MPTP+ Example 2 (50 mg/kg)

[FIG. 8]

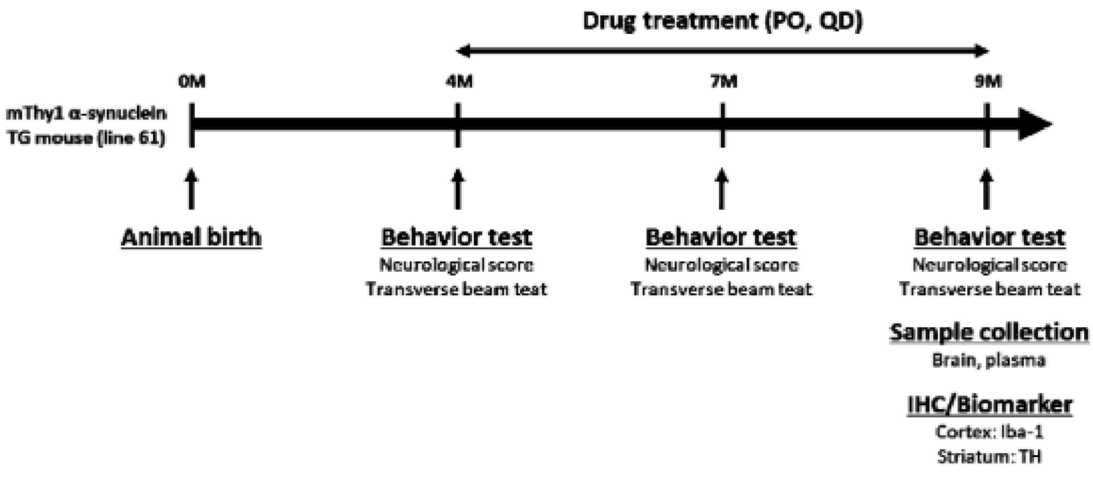
Drug treatment (PO, QD)
0M
4M
7M
9M
mThy1 α-synuclein TG mouse (line 61)
Animal birth
Behavior test
Neurological score
Transverse beam teat
Behavior test
Neurological score
Transverse beam teat
Behavior test
Neurological score
Transverse beam teat
Sample collection
Brain, plasma
IHC/Biomarker
Cortex: Iba-1
Striatum: TH

[FIG. 9]

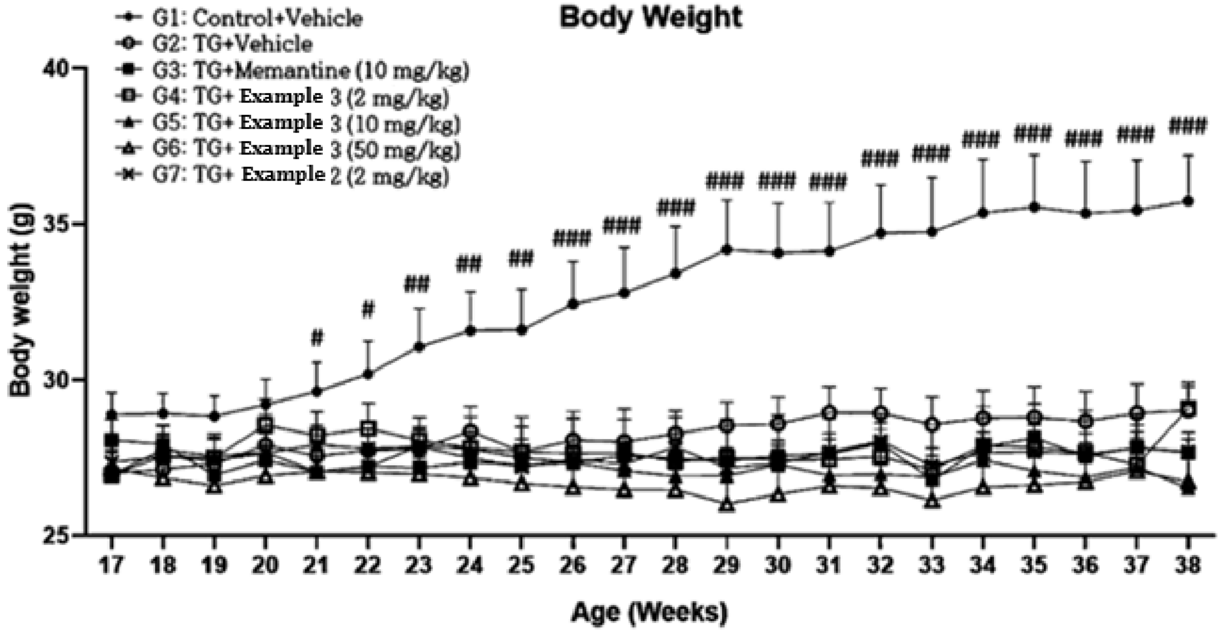


Statistical Significance

| | 17 | 18 | 19 | 20 | 21 | 22 | 23 | 24 | 25 | 26 | 27 | 28 | 29 | 30 | 31 | 32 | 33 | 34 | 35 | 36 | 37 | 38 |
|---|---|---|---|---|---|---|---|---|---|---|---|---|---|---|---|---|---|---|---|---|---|---|
| G2 | ns | ns | ns | ns | * | * | ** | ** | ** | *** | *** | *** | *** | *** | *** | *** | *** | *** | *** | *** | *** | *** |
| G3 | ns | ns | ns | ns | * | ** | *** | *** | *** | *** | *** | *** | *** | *** | *** | *** | *** | *** | *** | *** | *** | *** |
| G4 | ns | ns | ns | ns | ns | ns | ** | ** | ** | *** | *** | *** | *** | *** | *** | *** | *** | *** | *** | *** | *** | *** |
| G5 | ns | ns | ns | ns | * | ** | ** | ** | *** | *** | *** | *** | *** | *** | *** | *** | *** | *** | *** | *** | *** | *** |
| G6 | ns | ns | ns | ns | * | ** | *** | *** | *** | *** | *** | *** | *** | *** | *** | *** | *** | *** | *** | *** | *** | *** |
| G7 | ns | ns | ns | ns | ns | * | ** | *** | *** | *** | *** | *** | *** | *** | *** | *** | *** | *** | *** | *** | *** | *** |

[FIG. 10]

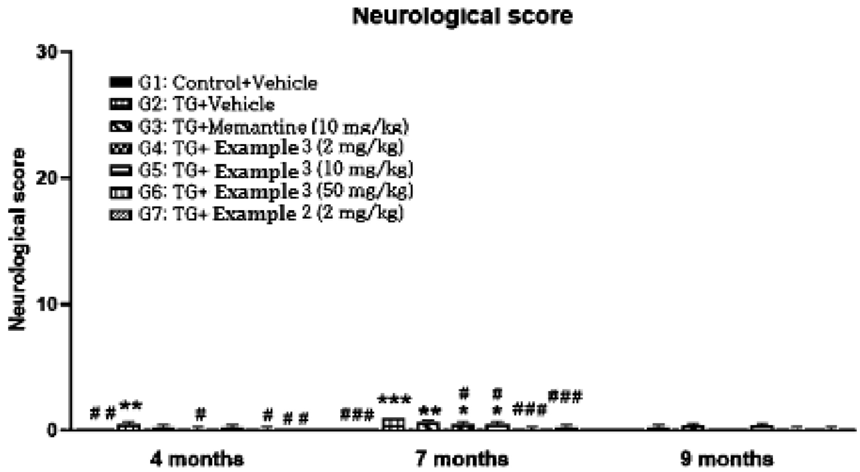

[FIG. 11]

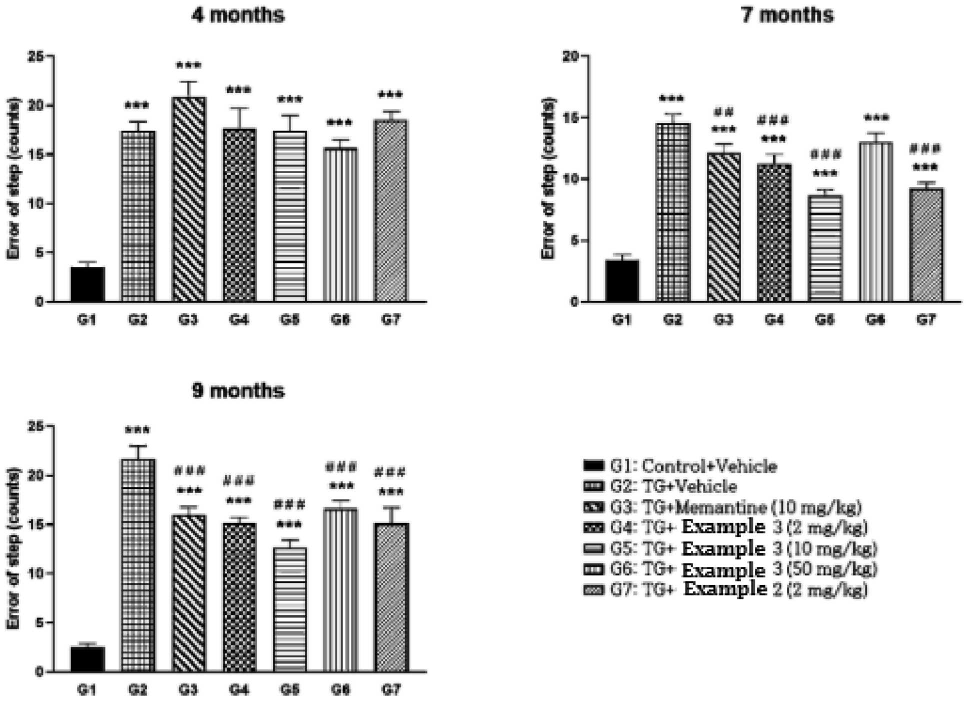


[FIG. 12a]

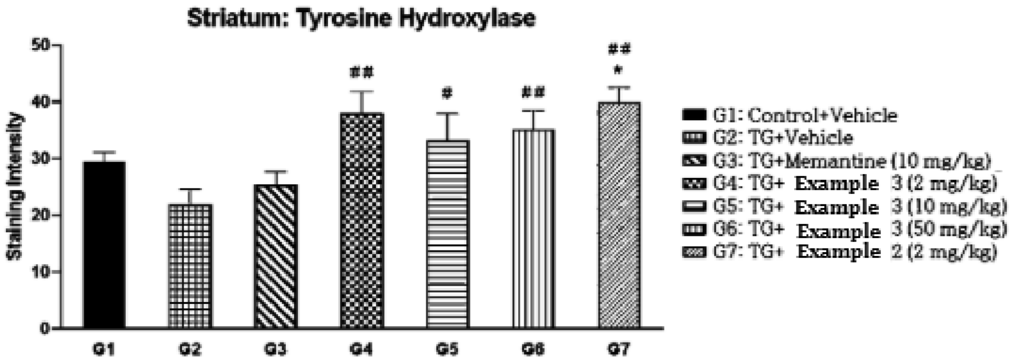

[FIG. 12b]

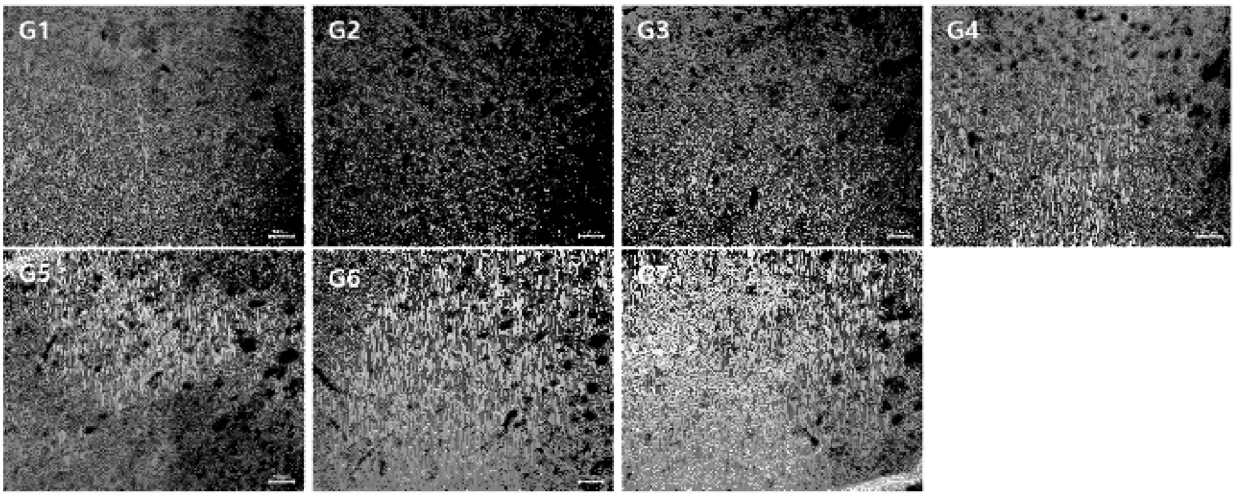


[FIG. 13a]

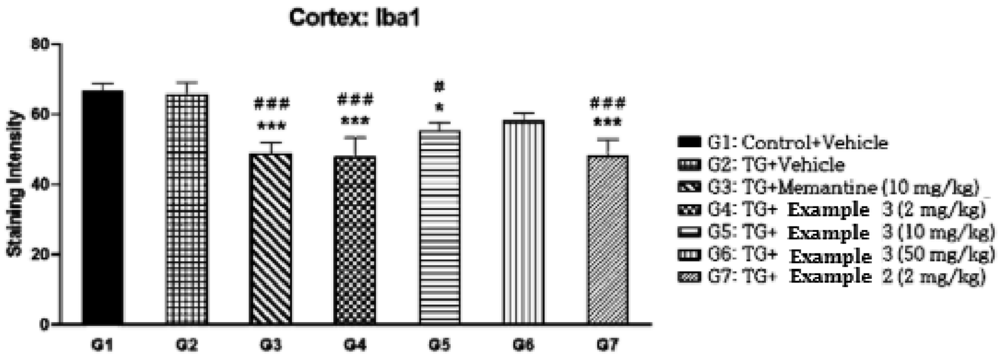

[FIG. 13b]

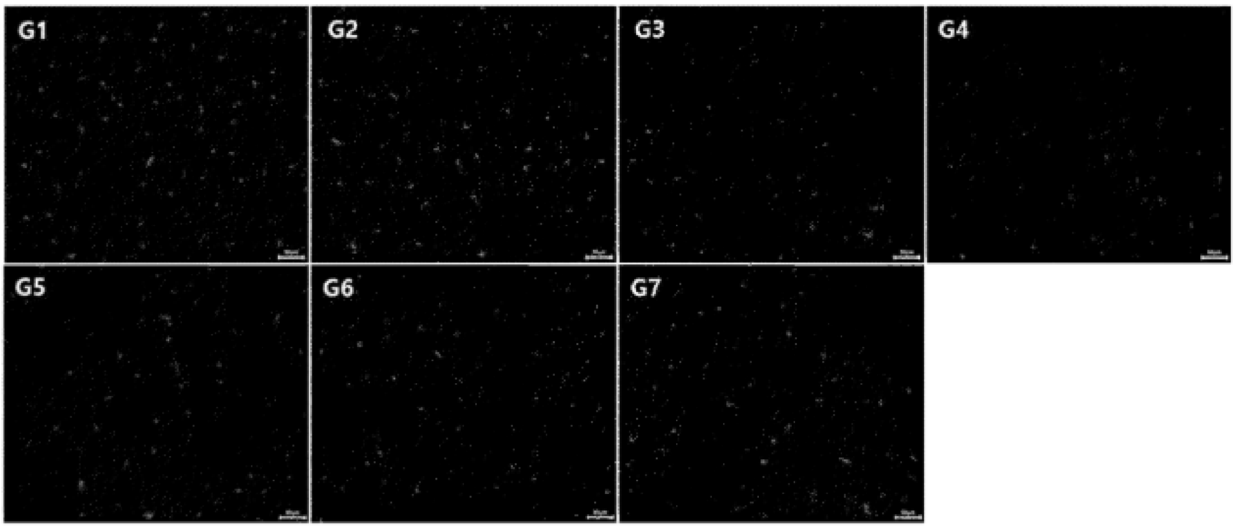


[FIG. 14]

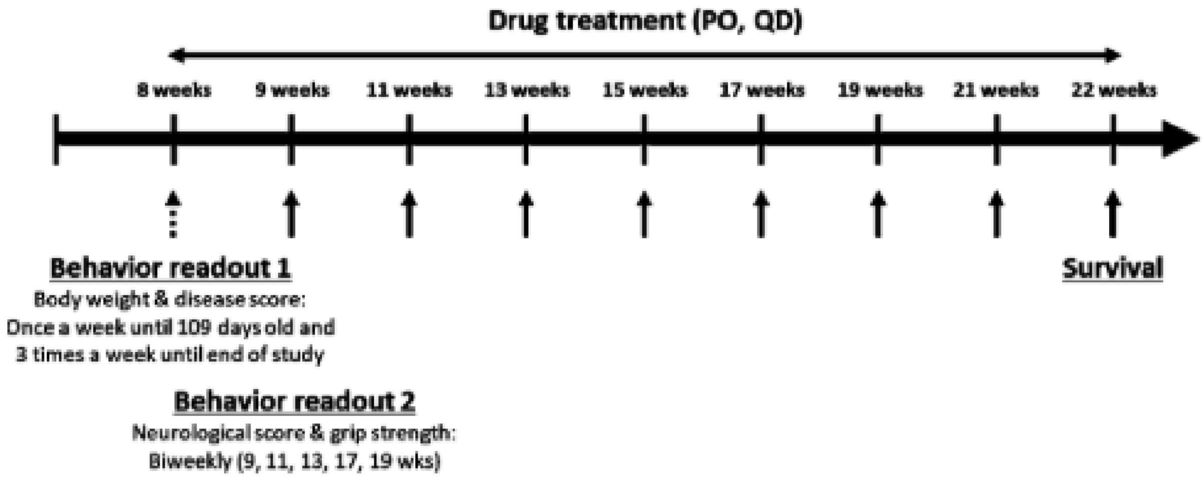

[FIG. 15]

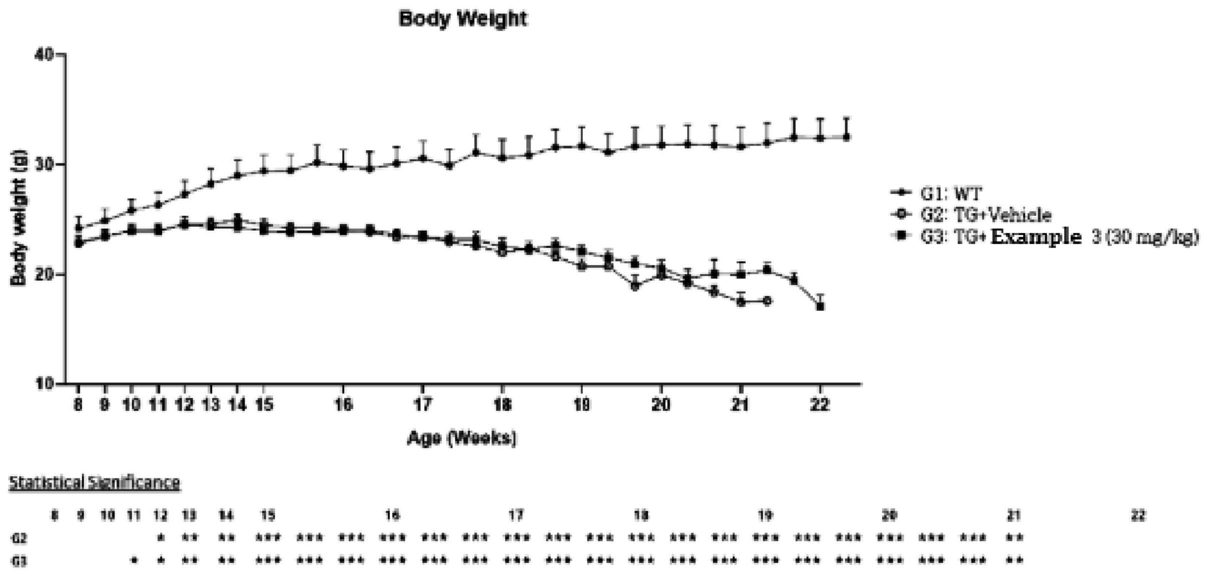
Body Weight
Body weight (g)
Age (Weeks)
G1: WT
G2: TG+Vehicle
G3: TG+ Example 3 (30 mg/kg)
Statistical Significance

[FIG. 16]

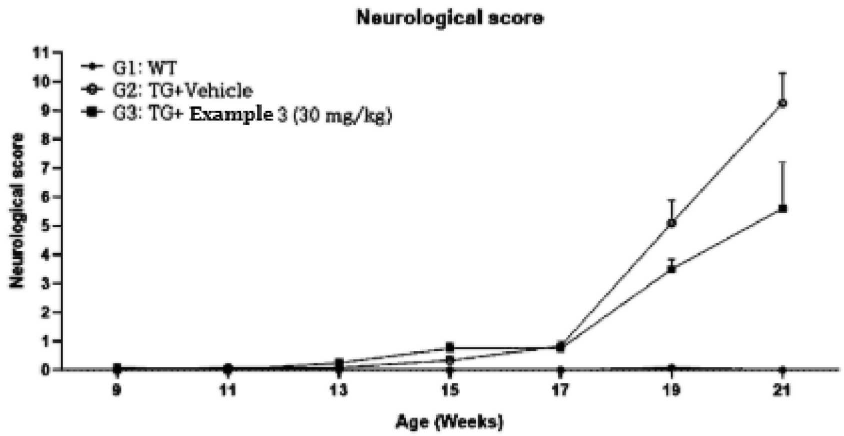
Neurological score
Neurological score
Age (Weeks)
G1: WT
G2: TG+Vehicle
G3: TG+ Example 3 (30 mg/kg)

[FIG. 17]

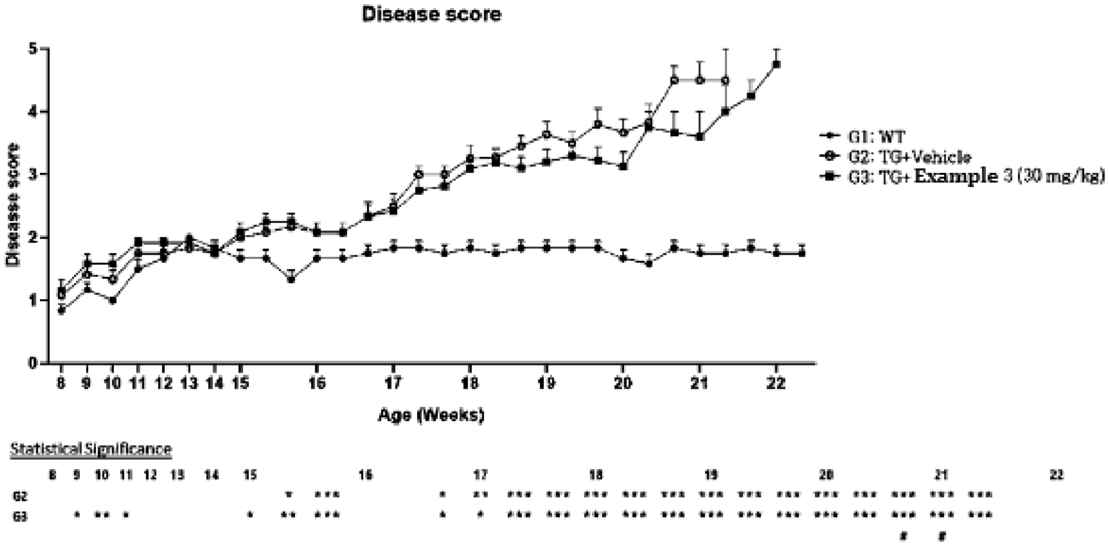


[FIG. 18]

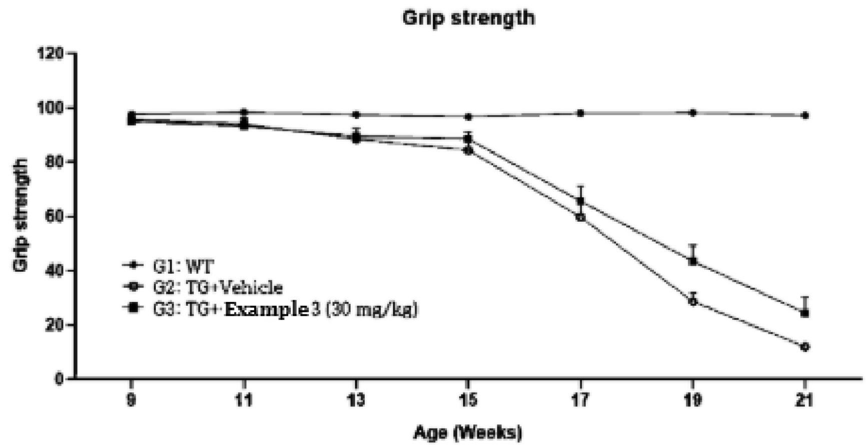


Statistical Significance

| | 9 | 11 | 13 | 15 | 17 | 19 | 21 |
|---|---|---|---|---|---|---|---|
| G2 | | | * | ** | *** | *** | *** |
| G3 | | | | * | *** | *** | *** |
| | | | | | | ## | ## |

[FIG. 19]

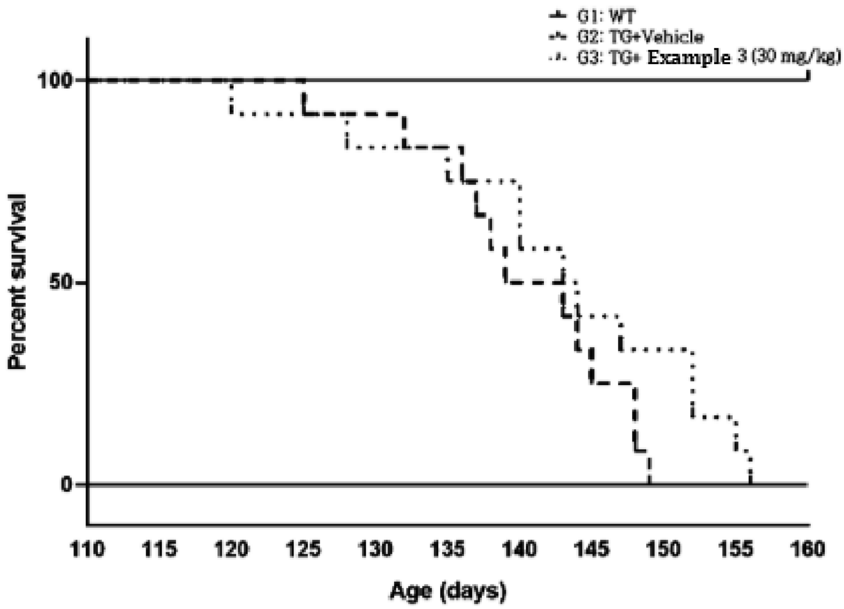
G1: WT
G2: TG+Vehicle
G3: TG+ Example 3 (30 mg/kg)
Percent survival
100
50
0
110
115
120
125
130
135
140
145
150
155
160
Age (days)

[FIG. 20]

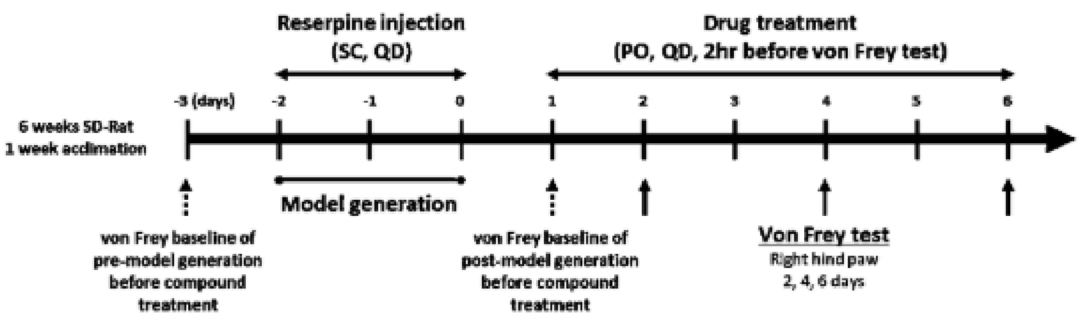
Reserpine injection
(SC, QD)
Drug treatment
(PO, QD, 2hr before von Frey test)
-3 (days)
-2
-1
0
1
2
3
4
5
6
6 weeks SD-Rat
1 week acclimation
Model generation
von Frey baseline of
pre-model generation
before compound
treatment
von Frey baseline of
post-model generation
before compound
treatment
Von Frey test
Right hind paw
2, 4, 6 days

[FIG. 21]

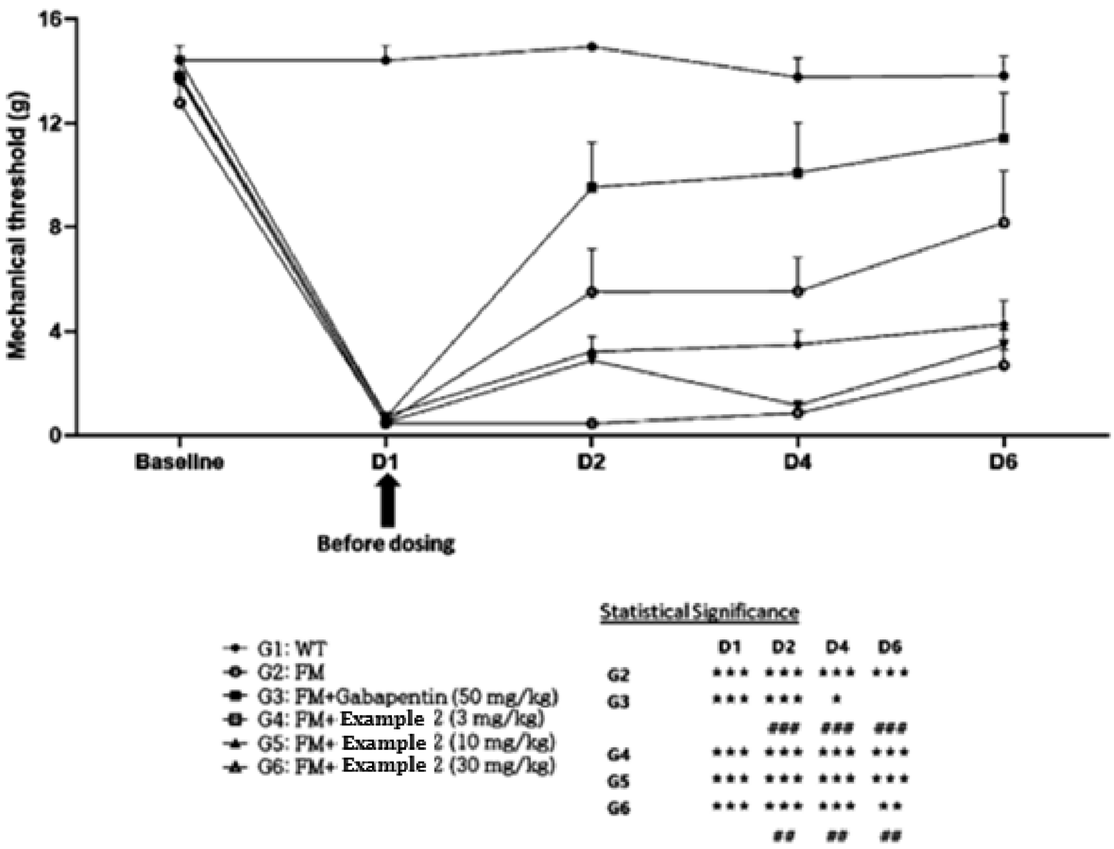
Mechanical threshold (g)
16
12
8
4
0
Baseline
D1
D2
D4
D6
Before dosing
G1: WT
G2: FM
G3: FM+Gabapentin (50 mg/kg)
G4: FM+ Example 2 (3 mg/kg)
G5: FM+ Example 2 (10 mg/kg)
G6: FM+ Example 2 (30 mg/kg)
Statistical Significance
D1 D2 D4 D6
G2 *** *** *** ***
G3 *** *** *
### ### ###
G4 *** *** *** ***
G5 *** *** *** ***
G6 *** *** *** **
## ## ##

[FIG. 22]

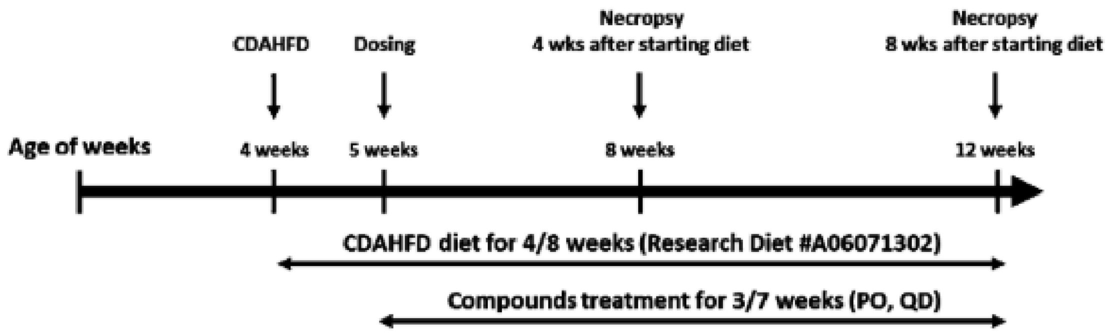
CDAHFD
Dosing
Necropsy
4 wks after starting diet
Necropsy
8 wks after starting diet
Age of weeks
4 weeks
5 weeks
8 weeks
12 weeks
CDAHFD diet for 4/8 weeks (Research Diet #A06071302)
Compounds treatment for 3/7 weeks (PO, QD)

[FIG. 23a]

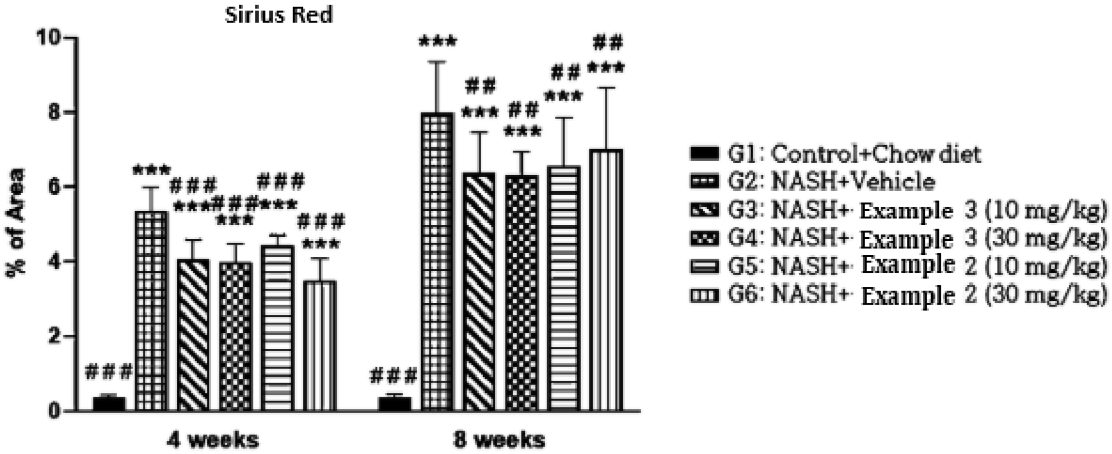


[FIG. 23b]

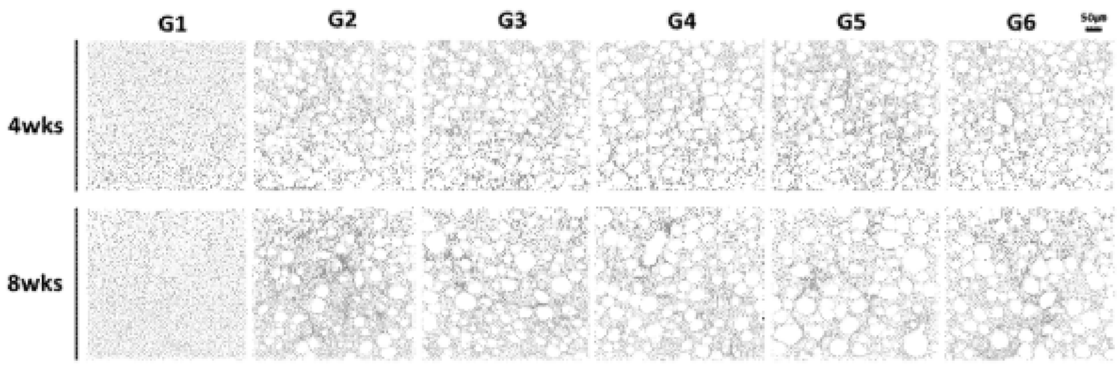

**INTERNATIONAL SEARCH REPORT**

International application No.
**PCT/KR2024/010056**

**A. CLASSIFICATION OF SUBJECT MATTER**

**C07D 401/14**(2006.01)i; **A61K 31/4439**(2006.01)i; **A61P 25/28**(2006.01)i; **A61P 9/00**(2006.01)i; **A61P 37/00**(2006.01)i; **A61P 1/16**(2006.01)i

According to International Patent Classification (IPC) or to both national classification and IPC

**B. FIELDS SEARCHED**

Minimum documentation searched (classification system followed by classification symbols)

C07D 401/14(2006.01); A61K 31/437(2006.01); A61K 31/4439(2006.01); A61P 37/06(2006.01); A61P 9/00(2006.01); C07D 471/04(2006.01); C07D 487/04(2006.01); C07D 491/107(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: ASK 1, 약학적 조성물 (pharmaceutical composition), 트리아졸 (triazole)

**C. DOCUMENTS CONSIDERED TO BE RELEVANT**

| Category* | Citation of document, with indication, where appropriate, of the relevant passages | Relevant to claim No. |
|---|---|---|
| A | WO 2020-030107 A1 (JIANGSU HANSOH PHARMACEUTICAL GROUP CO., LTD. et al.) 13 February 2020 (2020-02-13)<br>See claims 1, 31, 33 and 34. | 1-14,16,17 |
| A | WO 2020-015721 A1 (FUJIAN COSUNTER PHARMACEUTICAL CO., LTD.) 23 January 2020 (2020-01-23)<br>See claim 1. | 1-14,16,17 |
| A | CN 110922407 A (GUANGDONG HEC PHARMACEUTICAL) 27 March 2020 (2020-03-27)<br>See claim 1. | 1-14,16,17 |
| A | CN 110698471 A (XUANZHU PHARMA CO., LTD.) 17 January 2020 (2020-01-17)<br>See claims 1-8. | 1-14,16,17 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 October 2024** | **23 October 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/010056**

**C. DOCUMENTS CONSIDERED TO BE RELEVANT**

| Category* | Citation of document, with indication, where appropriate, of the relevant passages | Relevant to claim No. |
|---|---|---|
| A | CN 113831323 A (WUHAN RENFU INNOVATIVE DRUG R&D CENTER CO., LTD. et al.) 24 December 2021 (2021-12-24) See claim 5. | 1-14,16,17 |

Form PCT/ISA/210 (second sheet) (July 2022)

**INTERNATIONAL SEARCH REPORT**

International application No.
**PCT/KR2024/010056**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15**
because they relate to subject matter not required to be searched by this Authority, namely:

Claim 15 pertains to a method for treatment of the human body by therapy, as well as a diagnostic method (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2024/010056**

| Patent document cited in search report | | | Publication date (day/month/year) | Patent family member(s) | | | Publication date (day/month/year) |
|---|---|---|---|---|---|---|---|
| WO | 2020-030107 | A1 | 13 February 2020 | CN | 111107848 | A | 05 May 2020 |
| | | | | CN | 111107848 | B | 23 May 2023 |
| | | | | TW | 202012412 | A | 01 April 2020 |
| WO | 2020-015721 | A1 | 23 January 2020 | CN | 112638912 | A | 09 April 2021 |
| | | | | CN | 112638912 | B | 26 April 2022 |
| | | | | EP | 3825315 | A1 | 26 May 2021 |
| | | | | EP | 3825315 | A4 | 24 November 2021 |
| | | | | EP | 3825315 | B1 | 31 August 2022 |
| | | | | JP | 2021-531343 | A | 18 November 2021 |
| | | | | JP | 7096460 | B2 | 06 July 2022 |
| | | | | TW | 202019926 | A | 01 June 2020 |
| | | | | TW | I710561 | B | 21 November 2020 |
| | | | | US | 11814382 | B2 | 14 November 2023 |
| | | | | US | 2021-0269441 | A1 | 02 September 2021 |
| CN | 110922407 | A | 27 March 2020 | CN | 110922407 | B | 12 November 2021 |
| CN | 110698471 | A | 17 January 2020 | | None | | |
| CN | 113831323 | A | 24 December 2021 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013112741 A


### Non-patent literature cited in the description


- **ZHANG et al.** *J Clin Invest.*, 2003, vol. 111 (12), 1933-1943
- **FLEMING et al.** *J Neurosci*, 2004, vol. 24, 9434-40